# EUROPEAN PATENT SPECIFICATION

(11) **EP 1 752 541 B1**
(45) Date of publication and mention of the grant of the patent: **26.10.2011**
(21) Application number: 05748636.7
(22) Date of filing: 10.05.2005
(51) Int. Cl.: C12N 15/867, C12Q 1/70

(54) **NOVEL HIV-BASED RECOMBINANT VIRAL CLONES AND USE THEREOF IN ANALYTICAL METHODS**
NEUE REKOMBINANTE VIRUSKLONE AUF HIV-BASIS UND VERWENDUNG DAVON IN ANALYSEVERFAHREN
NOUVEAUX CLONES VIRAUX RECOMBINES BASES SUR LE VIH ET LEUR UTILISATION DANS DES METHODES ANALYTIQUES

(30) Priority: 10.05.2004 ES 200401116
(43) Date of publication of application: 14.02.2007
(73) Proprietor: Fundación para la Investigación y la Prevención del Sida en España, 28029 Madrid (ES); Instituto de Salud Carlos III, 28029 Madrid (ES)
(72) Inventor: ALCAMÍ PERTEJO, José, E-28009 Madrid (ES); GARCÍA PEREZ, Javier, E-28914 Leganés (Madrid) (ES); SÁNCHEZ PALOMINO, Sonsoles, E-08018 Barcelona (ES); GONZÁLEZ FERNÁNDEZ, Nuria, E-28011 Madrid (ES)
(74) Representative: Ungria Lopez, Javier
(86) International application number: PCT/ES2005/000250
(87) International publication number: WO 2005/108588

(56) References cited:
- WO-A2-01/79542
- US-A1- 2003 013 078
- PETROPOULOS ET AL: 'A novel phenotypic drug susceptibility assay for human immunodeficiency virus type 1.' ANTIMICROBIAL AGENTS AND CHEMOTHERAPY. vol. 44, no. 4, April 2000, pages 920 - 928, XP002962272
- MARTINEZ-PICADO J ET AL: 'Human immunodeficiency virus type 1 cloning vectors for antiretroviral resistance testing.' JOURNAL OF CLINICAL MICROBIOLOGY. vol. 37, no. 9, September 1999, pages 2943 - 2951, XP002933597
- DITTMAR M ET AL: 'A recombinant virus assay using full-length envelope sequences to detect changes in HIV-1 co-receptor usage.' VIRUS GENES. vol. 23, no. 3, 2001, pages 281 - 290, XP009022933

## Description

### TECHNICAL FIELD OF THE INVENTION

Within the broad field of research being conducted into AIDS and more specifically into the development of new families of drugs, the present invention focuses on the generation of certain new recombinant viral clones based on the genome of Human Immunodeficiency Virus (HIV) intended for being advantageously used in sensitivity tests to drugs, detection assays for neutralising antibodies, study of tropism and viral replicative capacity and methods of screening and characterisation of compounds with antiviral activity, etc.

### STATE OF THE ART PRIOR TO THE INVENTION

In the last five years the clinical evolution of patients infected with HIV has improved spectacularly thanks to the introduction of new families of antiretroviral drugs (Havlir and Lange, 1998), and as a consequence there has been a fall in the number of cases of AIDS, of the incidence of opportunistic infections and of mortality as a result of this disease.

Nevertheless, the successes achieved with those drugs have regrettably not made it possible to eradicate the disease since, in spite of the decrease in the plasmatic viral load to undetectable levels, viral replication persists at a low level in lymphoid organs (Chun et al., 1997; Finzi et al., 1997; Wong et al., 1997). Moreover, the proviral load, which reflects the pool of lymphocytes infected by HIV, does not decrease with antiretroviral treatment or it does so very slowly (Sharkey et al., 2000; Ramratnam et al., 2000). Finally, the suspension of antiretroviral medication leads to a rapid upturn in the viral load to base levels, even in patients that were found to be in apparently complete virological suppression (<5 copies of RNA/ml) for two years (Garcia et al., 1999). All this data suggests that the outlook for the eradication of AIDS with currently available medicines seems unlikely (Ho, 1998; Wein et al., 1998; Zhang et al., 1999; Furtado et al., 1999; Pomerantz, 1999). This possibility of eradication entails the development in the medium term of viruses resistant to the antiretroviral drugs used in each patient.

In this situation, a series of strategies against this disease continues to be underway, which can be summarised in the following points:
- Development of new drugs, and especially of new families of compounds with different targets from those currently considered by antiretroviral drugs.
- Development of therapeutic and preventive vaccines.
- Development of immunotherapy strategies aimed at strengthening the patient's immunological system.

Concomitant with the development of these strategies for fighting the disease, it is essential to develop analytical methods and techniques for evaluating these new approaches: models for determination of resistances to antiretrovirals, biological characterisation of qualitative aspects of the biology of the virus and development of models for the generation of platforms for screening and characterisation of the antiviral activity of the new compounds. In the following paragraphs, reference will be made to some of the analytical methods being used at present, and on which this invention has a special impact on account of its advantageous contributions.

### - Systems for determination of phenotypic resistances to antiretroviral drugs.

The determinations of phenotypic resistances is not done routinely in patients with HIV infection displaying virological failure, due to their extreme laboriousness and high cost. These tests on phenotypic resistances are usually done by a method selected from among one of the following two groups of systems:
a. Classical systems: In a first step, these consist on the isolation of the HIV starting from cultures of the patient's lymphocytes and, in a second step, infection of the target cells in the presence of different antiretrovirals in order to determine the inhibition concentration of the drugs (IC50) on a specific isolate. These systems are terribly expensive, lengthy, tedious and they require bio-security systems that are within the reach of very few virology laboratories (Richman et al., 1993, Nagy et al.; 1994).
b: Systems based on genetic recombination techniques. In this technology, the sequences of the pol gene are amplified on the basis of the patient's plasma and transfected together with the provirus selected in those sequences, in cell lines. By means of *in vivo* ligation reactions inside these cells, a virus carrying the Reverse Transcriptase and Protease sequences from the patient's virus is recombined. The recombinant viral progeny that is generated is used for evaluating the IC50 in the infection of target cells. There exist different variants of this technology in terms of the sequences and steps for amplification, target cells and use of markers (Boucher et al., 1996; Hertogs et al., 1998; Ruiz et al., 1998; Little et al., 1999; Borden et al., 1999). In spite of these developments which simplify the classical systems, testing techniques for viral recombination have limitations such as the low *in vivo* recombination rates, and it is still expensive and laborious.

Owing to its complexity and difficulties of standardisation, tests on phenotypic resistance to antiretroviral drugs are in practice available in a small number of laboratories and are essentially used for diagnostic purposes.

So, there exists a need for new techniques, simpler and more accessible, which would permit these determinations to be made in any laboratory, quickly, simply and economically.

### - Systems for the determination of the replicative capacity of HIV.

Among the qualitative characteristics to be found among the existing different isolates of HIV is "replicative capacity" or viral "fitness" (Ruiz Jarabo et al., 2002; Domingo, et al., 2001). Viral fitness is the final result of a multiple set of characteristics of the virus in the process of adaptation to its host. Nevertheless, in some situations, it has been seen that a diminished viral fitness is associated with the clinical evolution of the disease (Tersmette et al., 1995; Learmont et al., 1995). In particular, in a high percentage of long-term surviving patients it is extremely difficult to isolate their viruses in culture owing to their low replicative capacity (Cao et al., 1995; Pantaleo, et al., 1995; Michael et al., 1995). Perhaps of greater clinical relevance is the fact that viruses from multiresistant patients seem to replicate with a lower capacity (Mammano et al., 2000; Martinez-Picado et al.; 2000; Nijhuis et al., 2001; Spira et al., 2003).

The systems for determination of viral fitness are based on competition studies in culture between a wild virus and a virus displaying different mutations (Yuste et al., 1999; Iglesias et al., 2002). These methods require prolonged cultures and are therefore very laborious, expensive and difficult to standardise. The use of recombinant viruses for determining viral fitness has only recently been proposed (Deeka et al., 2001; Barbour et al., 2002) though this technique has not been properly standardised at the present time. With the aim of being able to assess in a precise way the replicative capacity of the virus, it is essential to be able to have techniques that are simple, reliable, accessible and rapid.

### - Systems for the detection of the presence of neutralising antibodies as an efficacy response parameter to experimental vaccines and immunomodulator treatments.

Infection by a virus induces a dual specific immune response in the host: activation of cytotoxic lymphocytes and production of antibodies (McMichael A., 2001; Burton DR., 2002). Of the latter, only those antibodies which block the entry of the virus in the target cell by various mechanisms possess efficacy in controlling the infection. This type of antibody is said to be "neutralising" and the importance of their role in HIV infection has been demonstrated by different works in recent years (Burton DR, 2002; Moore J and Burton DR, 1999).

The measurement of neutralising antibodies is important in a series of clinical situations since it has been shown that their presence is associated with a good prognosis for the infection (Cao et al., 1995; Lathey et al., 1997; Pilgrim et al., 1997; Lomig-Price, et al., 1998). Nevertheless, the greatest application of neutralising antibodies in the next few years will be taking place in the evaluation of new vaccines against HIV. There currently exists more than 50 preparations produced under GMP rules and 35 in phase I and II (McMichael AJ and Hanke T, 2003). In evaluating the efficacy of these preparations, the detection of neutralising antibodies will, together with cytotoxic activity against HIV, constitute the two parameters which will decide whether the preparation passes on to more advanced clinical study phases (Poignard et al., 1999; Moore JP and Burton DR.; 1999; McMichael AJ and Rowland Jones SL, 2001).

The neutralisation tests or tests for detection of neutralising antibodies are conducted by measuring the inhibition of cellular lysis by HIV in *in vitro* infection systems (Sattentau Q., 1996; Langlois et al., 1998).

This model has two important drawbacks:
a. An indirect effect of the viral replication is measured: that of cell destruction, but the replication of HIV is not measured directly.
b. The inhibition of a laboratory strain is analysed which means that antibodies against the specific virus of the patient are not detected, an aspect which can affect the characterisation of a specific response of the host.

Other techniques have been proposed based on microscopy or cytometry of infected cells but they entail a complexity that does not make them viable as routine tests (Haussmann et al., 1987; Mascola et el., 2002). The technique of infection inhibition by means of recombinant viruses has recently been introduced for analysing the neutralising capacity of serums in different experimental approaches (Kolchinski et al., 2001) and in clinical samples (Wei et al., 2003; Richman et al., 2003). It is therefore essential to develop new techniques for solving these two major drawbacks, permitting direct analysis of viral replication and its inhibition by the patient's antibodies, having high sensitivity and reliability and which can be conducted simply, quickly and economically.

### - Systems for the characterisation of viral tropism in HIV infection.

As well as the quantitative aspects of viral replication expressed by the plasmatic viral load, the different variants of HIV have a series of biological characteristics which characterise their pathogenicity. Among these, viral tropism, or the capacity of HIV to enter the cell via various receptors, is one of the most important viral characteristics (Weiss RA, 1996; Oberlin et al., 1997; Dorantz et al., 1996; Glushakova et al., 1998).

The existence of two larger receptors of HIV, known as CCR5 and CXCR4 (Loetscher et al., 2000) means that the different viral variants are classified into three categories: R5, X4 and R5X4 in line with their capacity to enter the cell by one of the two receptors exclusively or both receptors (Berger et al., 1998).

The measurement of viral tropism is not normally done as a diagnostic test but it does represent a highly useful parameter in certain areas of research. Nevertheless, the introduction of specific drugs into the entry having as their target one of the two receptors, CCR5 or CXCR4, means that a characterisation of the viral tropism of the patient before commencing treatment with regard to one of these targets can very likely be expected in the future (Lazzarin et al., 2003; Este JA, 2003; Zaitseva et al, 2003).

So, there exists a need to have systems permitting the characterisation of viral tropism in HIV infection in a patient, by means of techniques that are simple and accessible to any analysis laboratory, systems which are so far unavailable.

### - Experimental models permitting rapid screening of compounds with potential antiviral activity.

Current treatments do not permit a cure of HIV infection and so the development of new drugs is a priority in the context of research into AIDS (De Clercq et al., 2002). In essence, two sources of new drugs exist: derivatives of natural products, essentially coming from the plant kingdom, or those generated by combinatory chemistry starting from computer models or crystalline structures of the target molecule (Chu and Cutler, 1992; Jung et al., 2000; Knowles et al., 2003; Rudin et el., 2003; Agrafiotis et al., 2002).

In both cases, the molecule and its derivatives have to be characterised in terms of their toxicity and antiviral activity in a series of models which have to be robotisable in order to permit efficient screening since thousands of compounds have to be tested. There exist different systems currently used from the classical ones in which protection against the cytopathic effect of a reference virus is measured (Pauwels et al., 1987) or specific ones which analyse a certain target by means of biochemical tests (Hazuda et al., 2000; Cherepanov et al., 1997; Walters et al., 2003).

Nevertheless, there continues to exist a demand for screening systems which permit the development of robotisable models with which screening tests can be carried out on thousands of compounds in a way that is faster, more reliable, safer and cheaper (Federsel et al., 2003; Bleicher et al., 2003).

Special mention to be made here to the teachings of the Patent Application US2003013078, wherein two viral clones based on NL4.3 virus in which the nef gene has been substituted for the Renilla luciferase gene. These constructions are used for the screening of new antiviral compounds. In relation to its teachings, the present invention describes further in the following way:
- The introduction of the gene LacZ in concrete segments of the viral genome permits the cloning of those wished genomic fragments from the patients avoiding savage sequences from the receiving vector. This strategy allows the set-up of a great variety of viral clones entailing different patient sequences.
- This also allows the cloning of fragments from distinct viral subtypes, and from circulating isolates including those resistant to different anti-viral compounds. This ability enhances the sensibility of the tests and represents a great advantage in the screening of new compounds.

Also, Petropoulos et al., 2000, describes a susceptibility assay based on the use of a viral clone in which the env gene has been substituted for the luciferase gene, and that permits the cloning of both the protease and the reverse transcriptase genes. In relation to its teachings, the present invention describes further in the following way:
- As per in the previous reference, the introduction of the gene LacZ in different segments of the viral genome permits the cloning of those wished genomic fragments direct from the patients, thus avoiding savage sequences from the receiving vector.
- The cloning of the luciferase gene at the nef gene locus permits the generation of viral progenie using only one vector. The system of Petropoulos needs to perform a co-transfection with a second vector entailing the env gene, as the virus envelope gene is needed for viable virus.
- The present invention generates viruses able to replicate in multiple replication cycles, thus enhancing the sensibility of the test, whereas those teached by Petropoulos permits one replication due to its pseudo-type construction.

So, in view of the situation described above, the applicant has directed his investigative efforts towards the search for new recombinant viral clones, whose creation, identification and applications have allowed him to conclude the present invention, which represents a great advance in solving the problems and drawbacks mentioned above, as will easily be deduced from a thorough reading of the rest of this descriptive specification.

Provided below is a list of the complete bibliographical references that have been cited above in the foregoing paragraphs.
- Agrafiotis DK et al., Combinatorial informatics in the post-genomic ERA. Nat rev Drug Discov. 2002,1:227.
- Barbour JD, Wrin T, Grant RM, Martin JN, Segal MR, Petropouls CJ, Deeks SG. Evolution of phenotypic drug susceptibility and viral replication capacity during long-term virologic failure of protease inhibitor therapy in human immunodeficiency virus-infected adults. J Virol, 20002; 76: 11104-12.
- Berger EA, Doms RW, Fenyo EM, Korber BT, Littman DR, Moore JP, Sattentau QJ, Schuitemaker H, Sodroski J, Weiss RA. A new classification for HIV-1. Nature. 1998; 391. 240.
- Blair W, Spicer T. HIV-1 reporter visuses and their use in assaying anti-viral compounds: US2003013078.
- Bleicher KH. Hit and lead generation: beyond high-throughput screening. Nat Rev Drug Discov. 2003; 2:369.
- Borden D, Hurley A, Zhang, L et al., HIV-1 drug resistance in newly infected individuals. JAMA 1999; 262: 1135-41.
- Boucher CA, Keulen W, van Bommel T, Nijhuis M, de Jong D, de Jong MD, Schipper P, Back NK. Human immunodeficiency virus type 1 drug susceptibility determination by using recombinant viruses generated from patients tested in a cell-killing assay. Antomicrob Agents Chemother 1996; 40: 2404-9.
- Burton DR. Opinion: Antibodies, viruses and vaccines. Nat Rev Immunol. 2002; 2: 706-13.
- Cao Y, Qin L, Zhang L, Safrit J, Ho DD, Virologic and immunologic characterisation of long-term survivors of human immunodeficiency virus type 1 infection. N Engl J Med. 1995; 332: 201-8.
- Cherepanov. Mode of interaction of G-quartets with the integrase of HIV. Mol Pharmacol. 1997. 52: 771.
- Chu CK and Cutler HG (Eds.). Natural Products as Antiviral Agents. Ed. C.K., (1992), Plenum Press, N.Y.
- Chun TW, Carruth L, Finzi D et al. Quantification of latent tissue reservoirs and total body viral load in HIV-1 infection. Nature 1997; 37: 183-8.
- Chun TW, Stuyver L, Mizell SB, Ehler LA, Mican JA, Baseler M, Lloyd AL, Nowank MA, Fauci AS. Presence of an inducible HIV-1 latent reservoir during highly active antiretroviral therapy. Proc Natl Acad Sci U S A 1997 Nov 25; 94 (24) : 13193-7.
- De Clercq E. Strategies in the design of antiviral drugs. Nat Rev Drug Discov. 2002; 1: 13-25.
- Deeks SG, Wrin T, Liegler T, Hoh R, Hayden M, Barbour JD, Hellmann NS, Petropoulos CJ, McCune JM, Hellerstein NS, Grant RM. Virologic and immunologic consequences of discounting combination antiretroviral drug therapy in HIV-inflected patients with detectable viremia. N Engl J Med. 2001; 344: 472-80.
- Domingo E, Mas A, Yuste E, Pariente E, Sierra S, Gutierrez-Riva M, Menendez-Arias L. Virus population dynamics, fitness variations and the control of viral disease: an update. Prog Drug Res. 2001; 57: 77-115.
- Dorantz BJ, Rucker J, Yi Y, et al. A dual-tropic primary HIV-1 isolate that uses fusin and the beta-chemokine receptors CKR5, CKR3 and CKR2 as fusion coreceptors. Cell 1996; 35: 1149-1158.
- Este JA. Virus entry as a target for anti-HIV intervention. Curr Med Chem. 2003; 10: 1617-32.
- Federsel HJ. Logistics of process R&D: transforming laboratory methods to manufacturing scale. Nat Rev Drug Discov. 2003; 2: 654.
- Finzi D, Hermankova M, Pierson T, et al. Identification of a reservoir for HIV-1 in patients on highly active antiretroviral therapy. Science 1997; 278: 1295-300.
- Furtado M,. Callaways DS, Phair JP et al. Persistance of HIV transcription in patients receiving combination antiretroviral therapy. N. Engl. J. Med. 1999; 340: 1614-22.
- Garcia F, Plana M, Vidal C. et al. Dynamics of viral load rebound and immunological changes after stopping effective antiretroviral therapy. AIDS 1999; 13: f79.86.
- Glushakova S, Grivel JC, Fitzgerald W, Sylvester A, Zimmerberg J, Margolis LB. Evidence for HIV-1 phenotype switch as a causal factor in acquired immunodeficiency. Nature Med 1998; 4: 346-348.
- Hausmann EH, Gelderblom HE, Clapham PR, Pauli G, Weiss RA. Detection of HIV envelope specific antibodies by immunoelectron microscopy and correlation with antibody, titer and virus neutralizing activity. J Virol Methods. 1987; 16: 125-37.
- Havlir DV, Lange JM. New antiretrovirals and new combinations. AIDS 1998; 12 Suppl A:S165-74.
- Havlir DV, Marschner IC, Hirsch MS et al. Maintenance antiretroviral therapies in HIV infected patients with undetectable plasma HIV RNA after triple-drug therapy. AIDS Clinical Trials Group Study 343 Team. N Engl J Med 1998; 339: 1261-8.
- Hazuda DJ et al. Inhibitors of strand transfer that prevent integration and inhibit HIV-1 replication in cells. Science 2000; 287: 646.
- Hertogs K, de Bethune MP, Miller V et al. A rapid method for simultaneous detection of phenotypic resistance to inhibitors of protease and reverse transcriptase in recombinant human immunodeficiency virus type 1 isolates from patients treated with antiretroviral drugs. Antimicrob Agents Chemother 1998; 42: 269-76.
- Ho DD. Toward HIV eradication or remission: the tasks ahead. Science 1998; 280: 1866-7.

Iglesias-Ussel MD, Casado C, Yuste E, Olivares I, Lopez-Galindez C. In vitro analysis of human immunodeficiency virus type 1 resistance to nevirapine and fitness determination of resistant variants. J Gen Virol. 2002; 83): 93-101.
- Jung M. Recent studies on natural products as anti-HIV agents. Curr. Med. Chem. 2000; 649: 125.
- Knowles J. A guide to drug discovery: Target selection in drug discovery. Nat Rev Drug Discov. 2003; 2: 63.
- Kolchinsky P, Kiprilov E, Sodroski J. Increased neutralization sensitivity of CD4-independent human immunodeficiency virus variants. J Virol. 2001; 75: 2041-50.
- Lathey JL, Pratt RD, Spector SA. Appearance of autologous neutralizing antibody correlates with reduction in virus load and phenotype switch during primary infection with human immunodeficiency virus type 1 (Letter). J Infect Dis. 1997; 175: 231-2.
- Langlois AJ, Matthews TJ, Weinhold KJ, Chaffee S, Hershfield M, Bolognesi DP. Detection of HIV-1 neutralizing antibodies by a simple, rapid, colorimetric assay. AIDS Ras Hum Retrovisuses. 1988 Feb; 4(1): 63-9.
- Lazzarin A, Clotet B, Cooper D, Reynes J, Arasteh K, Nelson M, Katlama C. Stellbrink HJ, Defraissy JF, Lange J, Huson L, DeMasi R, Wat C, Delehanty J, Drobnes C, Salgo M; TORO 2 Study Group. Efficacy of enfuvirtide in patients infected with drug-resistant HIV-1 in Europe and Australia. N Engl J Med. 2003; 348: 2186-95.
- Learmont JC, Gezcy AF, Mills J, Ashton LJ, Raynes-Greenow CH, Garsia RJ, et al. Immunologic and virologic status after 14 to 18 years of infection with an attenuated strain of HIV-1. A report from the Sydney Blood Bank Cohort. N Engl J Med 1999; 340: 1715-22.
- Little SJ, Daar ES, D'Aquila RT et al. Reduced antiretroviral drug susceptibility among patients with primary HIV infection. JAMA 1999; 282: 1142-49.
- Loetscher P, Moser B, Baggiolini M. Chemokines and their receptors in lymphocyte traffic and HIV infection. Adv Immunol. 2000; 74: 127-80.
- Loomis-Price LD, Cox JH, Mascola JR, VanCott TC, Michael NL, Fouts TR et al., Correlation between humoral responses to human immunodeficiency virus type 1 envelope and disease progression in early-stage infection. J. Infect Dis. 1998; 178: 1306-16.
- Mammano F, Trouplin V, Zennou V, Clavel F. Retracing the evolutionary pathways of human immunodeficiency virus type 1 resistance to protease inhibitors: virus fitness in the absence and in the presence of drug. J Virol. 2000; 7: 8524-31.
- Martinez-Picado J, Savara AV, Shi L, Sutton L, D'Aquila RT. Fitness of human immunodeficiency virus type 1 protease inhibitor-selected single mutants. Virology. 2000 Sep 30; 275: 318 -22.
- Mascola JR, Louder MK, Winter C, Prabhakara R, De Rosa SC, Douek DC, Hill BJ, Gabuzda D, Roederer M. Human immunodeficiency virus type 1 neutralization measured by flow cytometric quantitation of single-round infection of primary human T cells. J Virol. 2002; 76: 4810-21
- McMichael AJ and Rowland-Jones SL. Cellular immune responses to HIV. Nature 2001; 410: 980-7.
- McMichael AJ and Hanke T. HIV vaccines 1998-2003; Nat. Med. 2003; 9: 874 -880.
- Michael NL, Chang G, d'Arcy LA, Ehrenberg PK, Mariani R, Busch MP, et al. Defective accessory genes in a human immunodeficiency virus type 1- infected long-term survivor lacking recoverable virus. J Virol. 1995; 69: 4228-36.
- Moore JP and Burton DR, HIV-1 neutralizing antibodies: how full is the bottle? Nat Med 1999; 5: 142-144
- Nagy K, Young M, Baboonian C, Merson J, Whittle P, Oroszlan S. Antiviral activity of human immunodeficiency virus type 1 protease inhibitors in a single cycle of infection: evidence for a role protease in the early phase. J Virol 1994; 68: 757-65.
- Nijhuis M, Deeks S and Boucher C. Implications of antiretroviral resistance on viral fitness. Curr. Opinion Inf. Dis. 2001; 14: 23-28
- Oberlin E, Amara A, Bachelerie F, et al. The CXc chemokine SDF-1 is the ligand for LESTR/Fusin and prevents infection by a T-cell line adapted HIV-1. Nature 1997; 382: 833-835.
- Pantaleo G, Menzo S, Vaccarezza M, Graziosi C, Cohen OJ, Demarest JF, et al. Studies in subjects with long-term nonprogressive human immunodeficiency virus infection. N Engl J Med. 1995; 332: 209-16.
- Pauwels, R et al. Sensitive and rapid assay on MT-4 cells for detection of antiviral compounds against the AIDS virus, J Virol Methods. 1987. 16: 171-185.
- Petropoulos CJ et al. A novel phenotypic drug susceptibility assay for human immunodeficiency virus type 1, Antimicrobial Agents and Chemotherapy. 2000; 44: 920-928.
- Pilgrim AK, Pantaleo G, Cohen OJ, Fink LM, Zhou JY, Zhou JT, et al. Neutralizing antibody responses to human immunodeficiency virus type 1 in primary infection and long-term-nonprogressive infection. J Infect Dis. 1997; 176: 924-32.
- Poignard P. Neutralizing antibodies have limited effects on the control of established HIV-1 infections in vivo. Immunity 1999; 10: 431-438.
- Pomeranzt RJ Residual HIV-1 disease in the era of HAART. N. Eng. J. Med. 1999; 340: 1625-27.
- Ramratnam B, Mitler JE, Zhang L. et al. The decay of the latent reservoir of replication-competent HIV-1 is inversely correlated with the extent of residual viral replication during prolonged antiretroviral therapy. Nat. Med. 2000; 6; 82-85.
- Richman DD, Johnson VA, Shirisaka T, O'Brien MC, Mitsuya H. Measurement of susceptibility of HIV-1 to antiviral drug. In: Strober W, Shevach E. eds. Current Protocols in Immunology. New York: Green Publishing Associates, 1993: 12.9.1.
- Richman DD, Wrin T, Little SJ, Petropoulos CJ. Rapid evolution of the neutralizing antibody response to HIV type 1 infection. Proc Natl Acad U S A. 2003; 100: 4144-9.
- Rudin M et al. Molecular imaging in drug discovery and development. Nat Rev Drug Discov. 2003; 2: 123.
- Ruiz L, Nijhuis M, Boucher C, Puig T, et al. Efficacy of adding indinavir to previous reverse transcriptase nucleoside in relation to genotypic and phenotypic resistance development in advanced HIV-1-infected patients. J Acquir Immune Defic Syndr Hum Retrovirol 1998: 19: 19-28.
- Ruiz-Jarabo CM, Arias A, Molina-Paris C, Briones C, Baranowski E, Escarmis C, Domingo E. Duration and fitness dependence of quasispecies memory. J Mol Biol. 2002; 315: 285-96.
- Sattentau Q. Neutralization of HIV-1 by antibody. Curr. Opin. Immunol. 1996; 8: 540-5.
- Sharkey ME, Teo I, Greenough T et al. Persistence of episomal HIV-1 infection intermediates in patients on highly active antiretroviral therapy. Nat. Med. 2000; 6: 76-81.
- Spira S, Wainberg MA,. Loemba H, Turner D, Brenner BG. Impact of clade diversity on HIV-1 virulence, antiretroviral drug sensitivity and drug resistance. J Antimocrob Chemother. 2003; 51: 229-40.
- Tersmette M, Lange JM, de Goede RE, de Wolf F, Eaftink-Schattenkerk JK, Schellekens PT, et al. Association between biological properties of human immunodeficiency virus variants and risk for AIDS and AIDS mortality. Lancet. 1989; 1: 983-5.
- Walters WF. Designing screens: how to make your hits a hit. Nat Rev Drug Discov. 2003; 2: 259.
- Wei X, Decker JM, Wang S, Hui H, Kappes JC, Wu X, Salazar-Gonzalez JF, Salazar MG, Kilby JM, Saag MS, Komarova NL, Nowak MA, Hahn BH, Kwong PD, Shaw GM. Antibody neutralization and escape by HIV-1. Nature. 2003; 422: 307-12.
- Wein L, D'Amato R, Perelson A. Mathematical analysis or antiretroviral therapy aimed at HIV-1 eradication or maintenance of low viral loads. J Theor Biol 1998; 192: 81-98.
- Weiss RA. HIV receptors and the pathogenesis of AIDS. Science 1996; 272: 1885-1886.
- Wong JK, Hezareh M, Günthard HF, Havlir DV, Ignacio CA, Spina CA and Richman D. recovery of replication competent HIV despite prolonged suppression of plasma viremia. Science 1997; 278: 1291-5.
- Yuste E, Sanchez-Palomino S, Casado C, Domingo E, Lopez-Galindez C. Related Articles, Links Drastic fitness loss in human immunodeficiency virus type 1 upon serial bottleneck events. J Virol. 1999; 73; 2745-51.
- Zaitseva M, Peden K, Golding H. HIV coreceptors: role of structure, posttranslational modifications, and internalization in viral-cell fusion and as targets for entry inhibitors. Biochim Biophys Acta. 2003; 1614: 51-61.
- Zhang I, Ramratnam B, Tenner-Racz K et al. Quantifying residual HIV-1 replication in patients receiving combination antiretroviral therapy. N Engl J Med. 1999; 340: 1605-13.

### DETAILED DESCRIPTION OF THE INVENTION

As stated in its title, this invention refers to the generation of new recombinant viral clones based on HIV and their use in analytical methods.

Within the context of the present invention, an HIV viral clone refers to a fragment of DNA containing all or practically all of the genome of the HIV including the two LTR of the proviral form of the virus. For the more specific case of HIV-1, the definition is the same, but substituting HIV for HIV-1.

The recombinant viral clones of the present invention are the result of a series of genetic manipulations made on said DNA fragment including deletion of viral genes, insertion of marker genes, introduction of mutations and substitution of genes or gene fragments from the original clone, with fragments from other clones or viral populations.

Specifically, the development of this invention has proceeded according to the following strategies:
- deletion of HIV fragments such as the *Nef* gene, so as to maintain the infective capacity of the recombinant viral clones that are generated;
- insertion into the proviral DNA of the marker gene *renilla,* a non-expressed gene in human cells. This enables the gene to function as a marker of infection, in other words, a cell which expresses renilla indicates that it has been infected;
- insertion of *LacZ* gene which codes for the enzyme Beta-galactosidase, substituting different sequences of the genome in order, on the one hand, to recognise the generation frequency of recombinant viruses and, on the other, to prevent dragging of wild viruses;
- introduction by directed mutagenesis of restriction sites which permit certain DNA fragments of the matrix provirus (such as for example Reverse Transcriptase, Protease, the complete *Pol* gene, *gag, nef* or the virus envelope) to be easily "extracted", so that they can be substituted with genes from isolates coming from patients to be assessed. This "cloning" system and generation of "chimera viruses" permits the characteristics of the different viral proteins of the patients to be studied in a system which presents all the advantages of marker genes.

The system of marking with renilla displays many advantages compared to the marker systems most commonly used nowadays, and it can be highlighted in particular that:
- the detection of renilla is a technique which has high sensitivity
- it can be used automatically and can even be robotised
- it is a cheap assay
- detection following infection with a virus carrying renilla as a marker is very fast (24 hours) compared to conventional systems for viral replication detection, which require between 5 days and a week of culture.

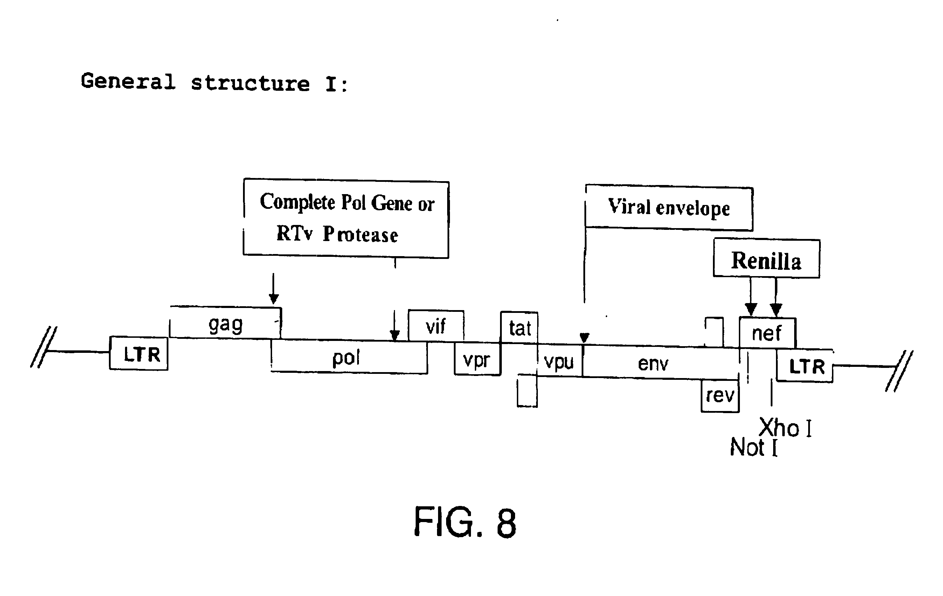

The HIV-based recombinant viral clones of the present invention are characterised in that they possess the general structure represented in figure 8, which contains the following elements in 5' to 3' direction:
- LTR or redundant terminal sequences (R) which contains numerous consensus sequences for transcription factor that regulate viral expression;
- *gag* is the gene which codes the p55 capsid protein formed by 3 protein subunits (MA, CA and Inc);
- *pol* is the gene which codes the viral enzymes needed for the viral replication process: protease (PRO), reverse transcriptase (RT) and integrase, and whose 5' end overlaps with *gag* element;
- *vif* is the gene that codes the protein Vif, it's 5' end overlaps with *pol* element and it's 3' end overlaps *vpr* element;
- *vpr* is the gene thatcodes the protein Vpr and it's 5' end overlaps *vif* element;
- tat is the gene that codes the protein Tat, it's second exon is contained inside *env* sequence;
- vpu is the gene that codes Vpu;
- *env* is the gene which codes the protein gp160 of the viral envelope;
- rev is the gene that codes the protein Rev, it's second exon is contained inside *env* sequence;
- *nef* is the gene that codes protein Nef, and is truncated at the bases in positions 8796 and 8887 of the viral genome;
- NotI is a restriction site for NotI enzyme, that has been introduced by directed mutagenesis at position 8796 of the viral genome;
- XhoI is a restriction site for the XhoI enzyme, in position 8887 of the viral genome;
- Renilla is the gene that codes the luciferase reporter protein Renilla, and that has been cloned in restriction sites NotI-XhoI in position 5' and 3', respectively; and
- LTR, whose 5' end overlaps with the 3' end of *nef* element.

In order to obtain the viral clones of the invention represented by the general structure (Figure 8), one starts from the proviral vector NL4.3 (Adachi A. Gendelman HE, Koenig S, Folks T, Willey R, Rabson A, Martin MA. Production of acquired immunodeficiency syndrome-associated retrovirus in human and nonhuman cells transfected with an infectious molecular clone. J. Virol. 1986 Aug; 59 (2): 284-91), which is genetically modified in the laboratory by means of different operations. Summarised below are the stages followed in the generation of the different viral clones. Given in bold and between brackets is the name of the intermediate and final gene constructions generated:
a) Introduction by directed mutagenesis of the NotI restriction site at the start of the nef gene (**IP NL Not**).
b) *Nef* gene deletion (cutting with restriction enzymes NotI and XhoI).
c) Cloning of the renilla gene in NotI/XhoI positions (**IP HIV NL Ren**). General structure (Figure 8).
d) Elimination of the unique NcoI site by means of digestion and filling with Klenow and introduction by directed mutagenesis of another NcoI restriction site in the position corresponding to amino acid 15 of retrotranscriptase, position 2593 of the DNA sequence, (change of glycine for alanine). **(IP HIV NL Nco Ren).**
e) Cloning in the **IP HIV NL Nco Ren** vector of the beta-galactosidase gene in the position of the RT **(IP HIV NL LacZ/rt Ren),** Protease **(IP HIV NL LacZ/pr Ren)** or the complete *pol* gene **(IP HIV NL LacZ/pol Ren)** with the aim of increasing the cloning efficacy and preventing dragging of minority populations of the reference virus. The clones religated without the patient's insert give blue colonies, while the plasmid that has incorporated the patient's RT, Pr or complete *pol* gene gives white colonies.
f) Starting from the **IP HIV NL LacZ/pr Ren** plasmid, destruction of the NarI restriction site external to the provirus by means of directed mutagenesis and introduction of the KspI restriction site in position 4498 by directed mutagenesis **(IP HIV NL LacZ/gag-pr Ren).**
g) Introduction by directed mutagenesis of the XbaI restriction site at position 6112 in clone **IP HIV NL Ren (IP HIV NL Xba Ren).**
h) Deletion of the envelope in plasmid **IP HIV NL XbaI Ren** by means of cutting with the restriction enzymes XbaI and NotI and cloning in its place of the *LacZ* gene **(IP HIV NL LacZ/env Ren**).
i) Generation of the viral clone **IP HIV JR Ren** cloning the envelope of the JR-CSF clone in the **IP HIV NL LacZ/Env Ren** plasmid.

The final vectors thus generated correspond to the new recombinant viral clones forming the object of this invention, all of them being included in the general structure (Figure 8). These viral clones have been deposited in the Spanish Collection of Type Cultures (University of Valencia, Burjassot, Valencia, Spain), in accordance with the rules of the Budapest Treaty on international recognition of deposited microorganisms for the purpose of patent procedure.

The particular structures of those viral clones are given below, indicated between brackets next to their name in the context of the present specification, is the name that has been assigned by the CECT:

### IP HIV NL Ren (CECT 5842)

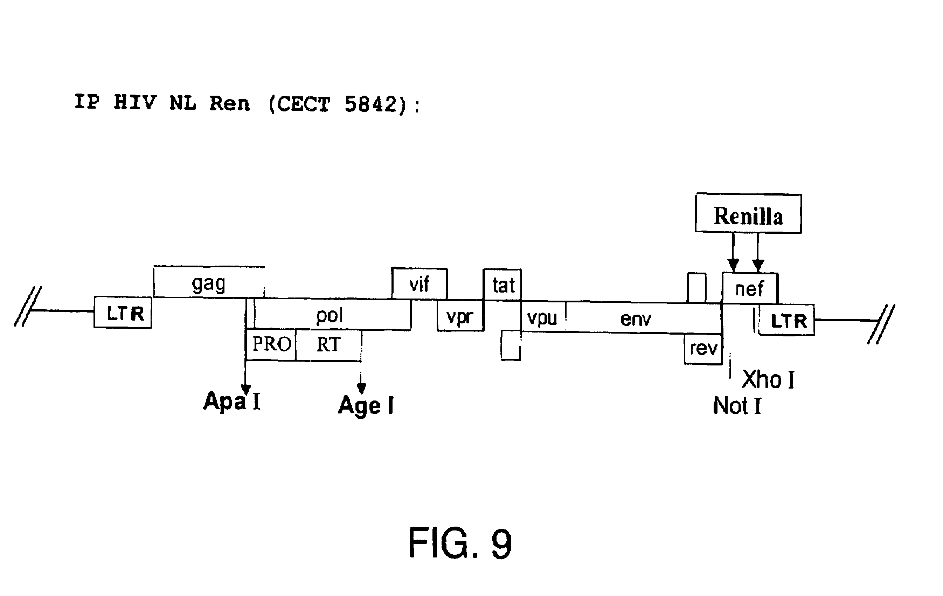

Recombinant viral clone based on the general structure previously described, characterized in that it possesses unique restriction sites for ApaI and AgeI enzymes introduced at positions 2006 and 3485, respectively, as shown in figure 9.

### IP HIV NL LacZ/pol Ren (CECT 5847)

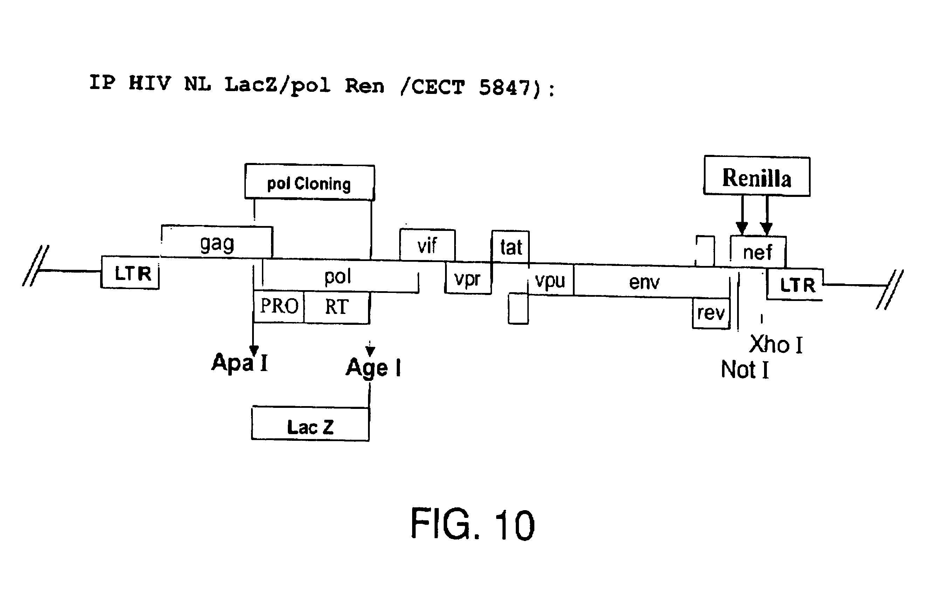

Recombinant viral clone based on the general structure previously described, characterized in that it possesses the *LacZ* gene cloned between restriction sites ApaI-AgeI in positions 5' and 3', respectively, substituting the fragment of *pol* gene that codes the protease and the reverse transcriptase, as shown in figure 10.

### IP HIV NL LacZ/pr Ren (CECT 5846)

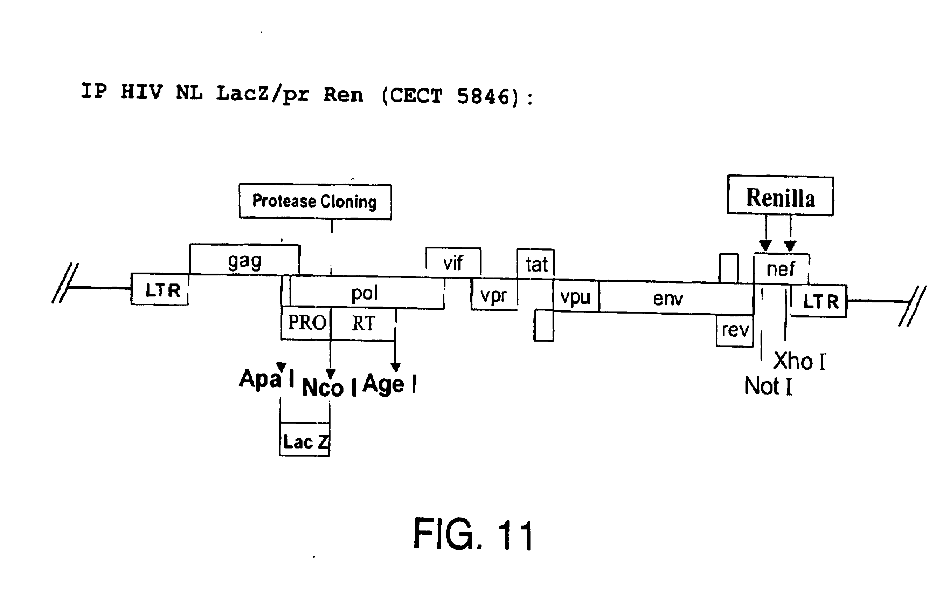

Recombinant viral clone based on the general structure previously described, characterized in that it possesses a unique restriction site for NcoI enzyme introduced by directed mutagenesis in position 2593 of the DNA sequence, and the *LacZ* gene cloned between restriction sites ApaI-NcoI in positions 5' and 3', respectively, substituting the fragment of the pol gene that encodes the protease, as shown in figure 11.

### IP HIV NL LacZ/rt Ren (CECT 5845)

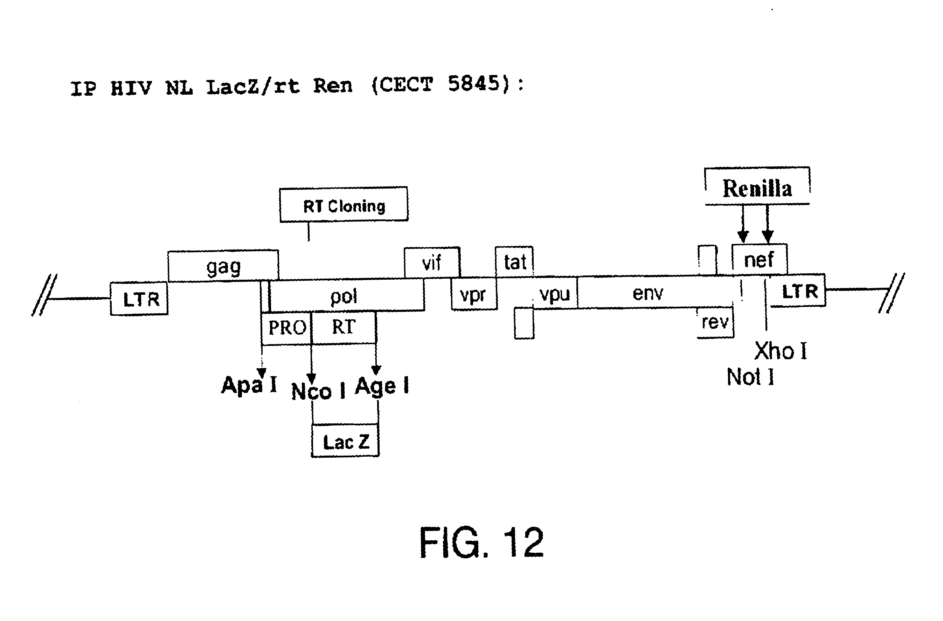

Recombinant viral clone based on the general structure described previously, characterized in that it possesses a unique restriction site for NcoI enzyme that has been introduced by directed mutagenesis in position 2593 of the DNA sequence, and the *LacZ* gene cloned between restriction sites NcoI-AgeI in position 5' and 3', respectively, substituting the fragment of *pol* gene that encodes the reverse transcriptase (Figure 12).

### IP HIV NL LacZ/gag-pr Ren (CECT 5848)

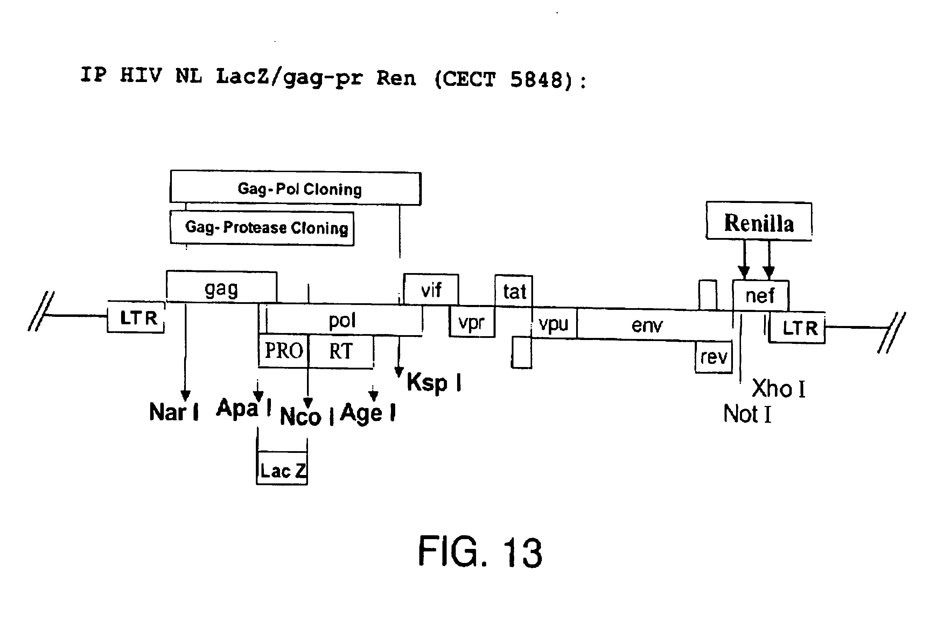

Recombinant viral clone based on the general structure previously described, characterized in that it possesses unique restriction sites, introduced by directed mutagenesis, for NarI and KspI enzymes in positions 637 and 4498 of the DNA sequence, respectively, and the *LacZ* gene cloned between restriction sites ApaI-NcoI in positions 5' and 3', respectively, substituting the fragment of *pol* gene that encodes the protease (Figure 13).

### IP HIV NL LacZ/env Ren (CECT 5844)

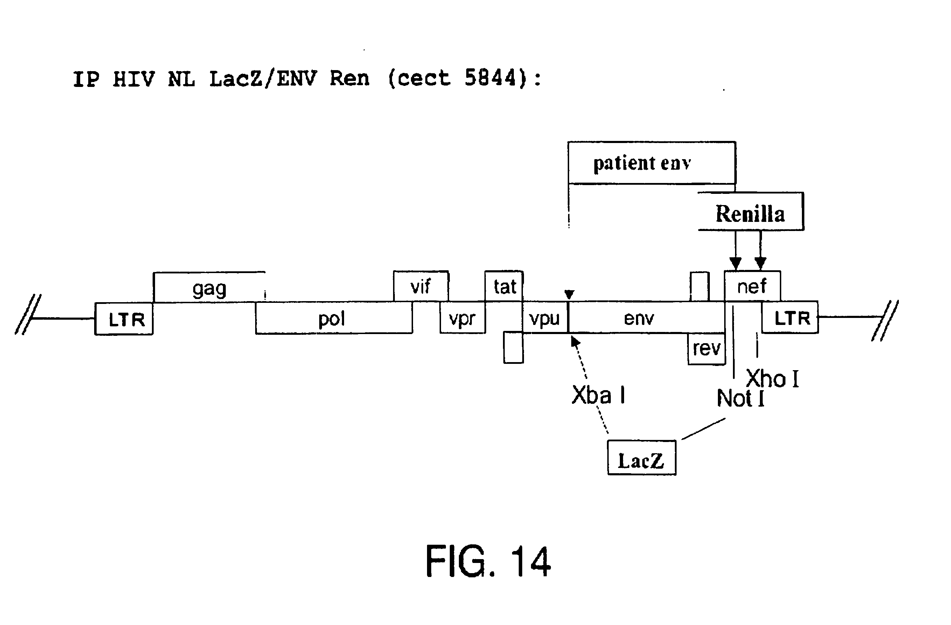

Recombinant viral clone based on the general structure previously described, characterized in that it possesses a unique restriction site for the XbaI enzyme introduced by directed mutagenesis in position 6112 of the DNA sequence, so as to allow the cloning of the envelope gene from the patient's virus, and also the LacZ gene cloned between restriction sites XbaI-NotI in positions 5' and 3', respectively, substituting *env* gene (Figure 14).

### IP HIV JRRen (CECT 5843)

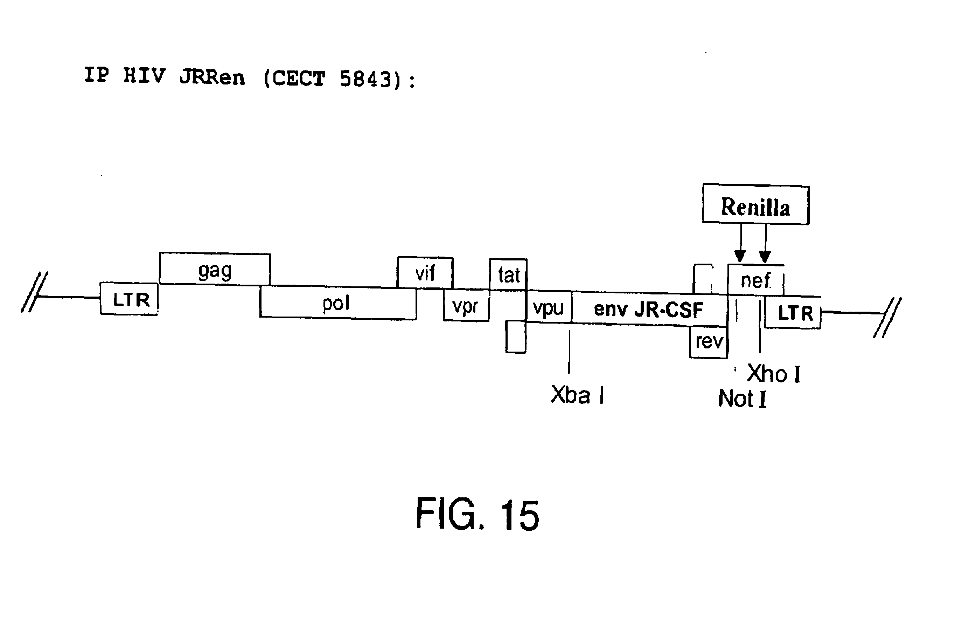

Recombinant viral clone based on the general structure previously described, characterized in that it possesses a unique restriction site for the XbaI enzyme introduced by directed mutagenesis in position 6112 of the DNA sequence; the *LacZ* gene cloned substituting *env* gene; and the gene *"env JR-CSF", env* gene from the clone JR-CSF, substituting the original *env* gene. This clone is represented in figure 15.

The recombinant viral clones of the present invention have shown themselves very useful in the development or improvement of analytical methods and techniques related to investigations surrounding AIDS. In fact, in the specific techniques that were described in the section on State of the Art, said clones have meant major advantages, some of which are detailed below:

### - Systems for determination of phenotypic resistance to antiretroviral drugs:

The proposed invention is based on the system of cloning HIV gene fragments of reverse transcriptase, of the envelope and of Protease into viral vectors that contain marker genes. This invention presents a series of advantages with respect to those already existing, namely:
a) The possibility of separately analysing the resistance to inhibitors of Protease, of Reverse Transcriptase and of the envelope. This makes it possible to perform an independent evaluation of resistances to different pharmacological groups.
b) the use of multiple cycle viral systems.
c) A greater efficacy in the evaluation of viral isolates with low replicative capacity.

### - Systems for determination of HIV replicative capacity:

The proposed invention permits this parameter to be determined and a direct analysis to be made of the viral replicative capacity in target cells very close to physiological targets such as peripheral blood lymphocytes. The cloning of the envelope genes and different fragments of the patient's *gag-pol* DNA in multiple cycle carrier viruses of marker genes (Renilla) confers the chimera virus with the replicative properties of the mutated virus. Unlike the evaluation systems for viral fitness, which are extremely laborious, the development permits analysis of the replicative capacity of the recombinant virus in a manner that is virtually continuous.

### - Systems for determination of the presence of neutralising antibodies:

The proposed invention permits the two main drawbacks of classical techniques for determining neutralising capacity in the serum of seropositive patients to be overcome, since it enables a direct analysis to be made of viral replication and its inhibition by the patient's antibodies. It is possible to do this both on isolates or reference viral clones, as well as on a recombinant virus in which the envelope of the viral clone has been substituted by the complete envelope of the patient's viral population.

This type of assay, known by the applicant as "autologous test for detection of neutralising antibodies", has a high sensitivity and allows a precise evaluation of the neutralising capacity of the patient's serum towards the viruses that are replicating in his organism at the moment of the test.

### - Systems for the characterisation of viral tropism in infection by HIV:

The proposed invention permits this parameter to be determined by means of two tools: the generation of recombinant viruses which carry the complete envelope of the patient's viral population and the use of a target cell which stably expresses both receptors (SSPA-B7).

### - Experimental models for the screening of compounds with potential antiviral activity:

The proposed invention permits the detection of antibody activity to be carried out in an easily robotisable microplate format, in a model which covers the entire viral replicative cycle by means of using multiple cycle vectors. In relation to the antiviral activity determination systems that currently exist, which assess the protection from the cytopathic effect, the proposed system permits an analysis of the direct inhibition of HIV replication and considerably cuts down on screening times.

Thus, the present invention also relates to the use of the previously described viral clones, in analytical methods for the determination of phenotypic resistances to antiretroviral compounds for treatment of HIV infection.

A specific realization of the invention refers to the use of said viral clones in analytical methods for the determination of the replicative capacity of the recombinant virus that carry the *gag, pol and*/*or env* sequences from patients with HIV infection.

On the other hand, the present invention relates to the use of said recombinant viral clones in analytical methods for the characterization of viral tropism in HIV infection.

In another particular embodiment, the present invention realtes to the use of said recombinant viral clones in analytical methods for the detection of neutralizing antibodies against HIV in the serum of patients seropositive for HIV and non-infected individuals, subjected to vaccination or otherwise.

Lastly, the present invention refers to the use of said recombinant viral clones in analytical methods for the screening and characterization of compounds with antiviral activity against HIV.

### BRIEF DESCRIPTION OF THE FIGURES

**Figures 1a** **and** **1b****:** Illustrative diagrams corresponding to the production of viral clones of the present invention, in accordance with the process described in preferred embodiment 1.
**Figures 2a** **and** **2b****:** Graphic representations corresponding to the results of studies discussed in section 2.1 of Modes of Embodiment of the Invention.
**Figure 3****:** Graphic representation corresponding to the determination studies of replicative capacity discussed in section 2.2 of Modes of Embodiment of the Invention.
**Figure 4****:** Expression of CCR5 and CXCR4 by the SSPA-B7 clone in accordance with section 2.3 and 2.4 of Modes of Embodiment of the Invention.
**Figure 5****:** Cytopathic effect induced in the clone SSPA-B7 by the isolates NL4.3 (X4) and Bal (R5), in accordance with sections 2.3 and 2.4 of Modes of Embodiment of the Invention.
**Figure 6****:** Analysis of the neutralising capacity of the NL-Luc virus of a patient's plasma under the conditions of section 2.4(D) of Modes of Embodiment of the Invention.
**Figure 7****:** Results of the analysis of antiviral activity of two compounds studied according to section 2.5(C) of Modes of Embodiment of the Invention.
**Figure 8****:** General structure of recombinant viral clones of the present invention, where: LTR (long terminal repeats) are the regions with redundant sequence (R) which plays a primary role during the retrotranscription process; *gag* is the gene which codes for the p55 protein of the capsid formed by 3 protein subunits (MA, CA and NC); *pol* is the gene which encodes the viral enzymes necessary for the viral replication process: protease (PRO), reverse transcriptase (RT) and integrase; *vif* codes the protein Vif associated with the infectiousness of the extracellular virions; *vpr* codes the Vpr protein which acts as the accelerator of the replication cycle at different levels; *tat* codes the protein Tat which is a transactivator; *vpu* encodes Vpu involved in the virions release; *env* is the gene which codes the protein gp160 of the viral envelope; rev produces the protein Rev, in charge of the processing and transport of messenger RNA to the cytoplasm; *nef* codes the protein Nef which negatively regulates CD4 and HLA molecules of the infected cell and plays a role in the pathogenicity of the virus; NotI and XhoI indicate unique restriction sites in the DNA sequence; Renilla indicates the cloning position of the reporter gene.
**Figure 9****:** Recombinant viral clone IP HIV NL Ren, deposited in the Spanish Collection of Type Cultures as CECT 5842, where ApaI and AgeI represent unique restriction sites in the DNA sequence and the remaining symbols have the meaning given above for Figure 8.
**Figure 10****:** Recombinant viral clone IP HIV NL LacZ/pol Ren, deposited in the Spanish Collection of Type Cultures as CECT 5847, where LacZ indicates the cloning position of the gene LacZ substituting a fragment of the *pol* gene, and the remaining symbols have the meaning given above.
**Figure 11****:** Recombinant viral clone IP HIV NL LacZ/pr Ren, deposited in the Spanish Collection of Type Cultures as CECT 5846, where NcoI indicates a unique restriction site in the DNA sequence, and the remaining symbols have the meaning given above.
**Figure 12****:** Recombinant viral clone IP HIV NL LacZ/rt Ren, deposited in the Spanish Collection of Type Cultures as CECT 5845, where the different symbols have the same meaning as above.
**Figure 13****:** Recombinant viral clone IP HIV NL LacZ/gag-pr Ren, deposited in the Spanish Collection of Type Cultures as CECT 5848, where NarI and KspI indicate unique restriction sites in the DNA sequence, and the remaining symbols have the meaning given above.
**Figure 14****:** Recombinant viral clone IP HIV NL LacZ/env Ren, deposited in the Spanish Collection of Type Cultures as CECT 5844, where XbaI indicates a unique restriction site in the DNA sequence, "patient env" indicates the cloning position of the patient's gene, and the remaining symbols have the meaning given above.
**Figure 15****:** Recombinant viral clone IP HIV JRRen, deposited in the Spanish Collection of Type Cultures as CECT 5843, where XbaI indicates a unique restriction site in the DNA sequence, "env JR-CSF" indicates the cloning position of the env gene of the clone JR-CSF in place of the envelope of NL 4.3 and the remaining symbols have the meaning given above.

### MODES OF EMBODIMENT OF THE INVENTION

The present invention is illustrated forthwith by means of a detailed description of preferred embodiments, in which the recombinant viral clones of the invention are shown along with the main applications together with some of the general techniques of genetic engineering used in the different cases, all this making use of the attached figures for greater clarity.

### 1.- OBTAINING OF RECOMBINANT VIRAL CLONES

This is based on the system of cloning gene fragments corresponding to HIV reverse transcriptase and protease in viral carrier vectors that contain marker genes.

### (A) General description of the technique:

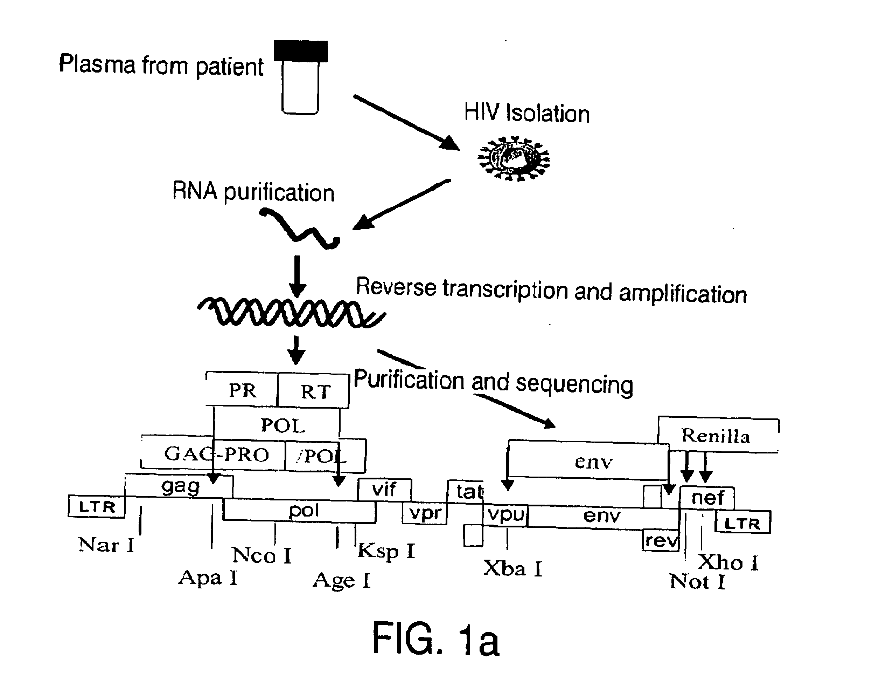

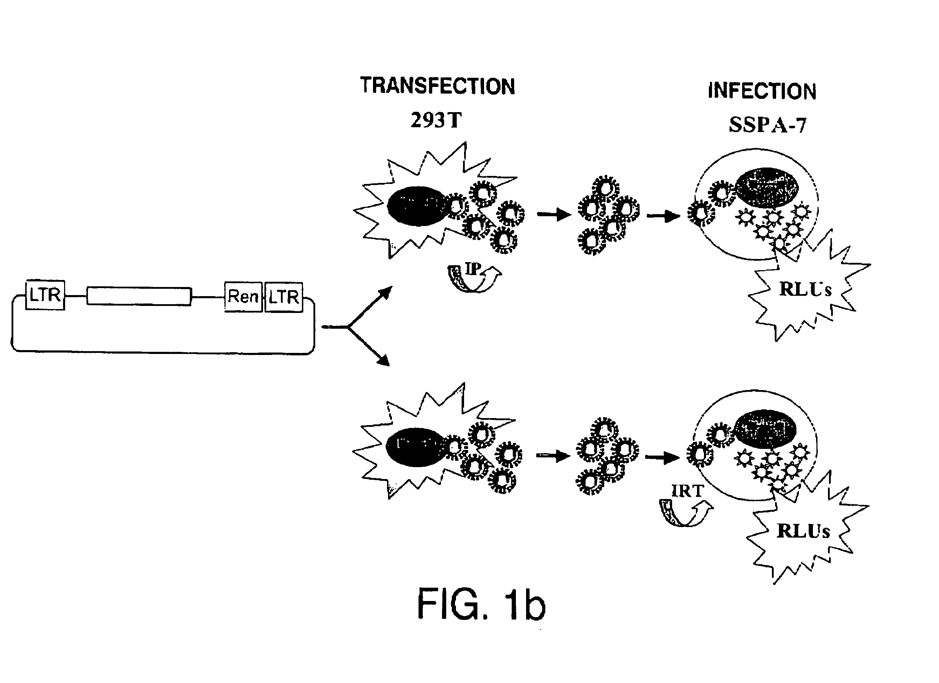

It can be schematically seen in Figure 1a and 1b how the viral particles are produced during the 48 hours following transfection of the viral plasma in 293T cells. The 293T cell line was obtained from the Deposit of the ATCC. The SSPA-B7 clone was obtained by the applicant from the MT-2 cell line by means of transfection of an expression vector of the gene CCR5 provided with a resistance marker for Genetycin. Following transfection, the supernatants are gathered and the SSPA-B7 target cells are infected. The capacity of the viruses to complete a replication cycle is quantified by measuring the luciferase activity in the target cells. The activity of the inhibitors of the protease is measured by adding them to the transfected cells while activity towards inhibitors of reverse transcriptase and of entry is measured by adding the drugs to the infected cells.

The process comprises the following operations:
- Starting from 0.5 ml of the patient's plasma, the extraction of RNA from the HIV is carried out.
- The viral RNA is retrotranscribed and then amplified using specific primers for each viral gene by means of polymerase chain reaction. The primers include specific restriction sites for later cloning into the reference virus, the *pol* gene or its fragments, or of the *env* gene in the different viral clones depending on the type of recombinant virus it is wished to generate.
- Following enzymatic digestion of the amplificate and of the reference virus, an *in vitro* ligation process is carried out using the T4 ligase.
- The population of the generated recombinant provirus is transfected in the 293T cell line and acts as a producer cell of recombinant viruses.
- The infectious progeny of recombinant viruses is gathered 48 hours after the transfection and is used for infecting the SSPA-B7 cell line.
- When the application is the determination of resistance to antiretrovirals, the last two processes are carried out in the presence of protease inhibitors (in the case of 293T producer cells), reverse transcriptase inhibitors (in the case of SSPA-B7 target cells), or viral entry inhibitors (in the case of SSPA-B7 target cells).
- The level of sensitivity of the different drugs is defined by means of the concentration that gives a 50% inhibition of viral replication (IC50) in comparison with a reference virus without any associated resistance mutations.
- The reading of the sensitivity to the different drugs is done by quantifying the renilla activity by means of a Berthold Orion Microplate luminometer.

### (B) Virus:

This starts from the proviral vector NL4.3 (Adachi et al. 1986). This clone has been genetically modified in the laboratory producing multiple cycle viral clones which express the indicator gene Renilla instead of nef and in which different restriction targets have been introduced in order to be able to clone the complete *pol* gene, the fragments Reverse Transcriptase or Protease separately, the regions *gag* protease and *gag-pol* or the complete *env* gene**.**

The recombinant viral clones obtained permit cloning of the patient's complete *pol gene,* the reverse transcriptase and protease separately, the *gag* region along with the protease or the complete *pol* gene. It also permits cloning of the patient's complete *env* gene. All these are multiple cycle viruses and are very useful when multiple resistance mutations in the patient's RT and Protease exist, as the final replicative capacity is improved.

### (C) Primers:

In the most important operations mentioned earlier, the following primers and the following cells are used:
- *Mutagenesis*
   Mutagenesis Not I:
   5' GCTATAAGATGGGTGGCGCGGCCGCAAAAAGTAGTGTGATTGG 3'
   5' CCAATCACACTACTTTTTGCGGCCGCGCCACCCATCTTATAGC 3'
   Mutagenesis Nco I:
   5' CCAGTAAAATTAAAGCCAGCCATGGATGGCCCAAAAG 3'
   5' CTTTTGGGCCATCCATGGCTGGCTTTAATTTTACTGG 3'
   Mutagenesis Ksp I:
   5' GAAGCAGAAGTAATTCCCGCGGAGACAGGGCAAGAAAC 3'
   5' GTTTCTTGCCCTGTCTCCGCGGGAATTACTTCTGCTTC 3'
   Mutagenesis Nar I:
   5' GAAAATACCGCATCAGGACCCATTCGCCATTCAGGC 3'
   5' GCCTGAATGGCGAATGGGTCCTGATGCGGTATTTTC 3'
   Mutagenesis Xbal:

5′ GCATTAGTAGTAGCAATAATAATAGCTCTAGAGCTGTGGTCCATAGTA
ATCATAG

5' CTATGATTACTATGGACCACAGCTCTAGAGCTATTATTATTGCTACTA
CTAATGC

### - Amplification of the pol gene of patients

POL: 5'GCCAAAAATTGCAGGGCCCCTAGG A 3.'
   5' TCTTTTGATGGGTCATAATACACTCCATGTACCGG 3'
PRO: 5' GCCAAAAATTGCAGGGCCCCTAGGA 3'
   5' CATGCCATGGCTGGCTTTAATTTTACTGGTACAGTC 3'
RT: 5' CATGCCATGGATGGCCCAAAAGTTAAACAATGGCC 3'
   5' TCTTTTGATGGGTCATAATACACTCCATGTACCGG 3'
GAG-PR: 5' GGAAAATCTCTAGCAGTGGCGCCCGAACAG 3'
   5' CATGCCATGGCTGGCTTTAATTTTACTGGTACAGTC 3'
GAG-POL: 5' GGAAAATCTCTAGCAGTGGCGCCCGAACAG 3'
   5' CTTGCCCTGTCTCTGCTGGAATTACTTCTGC 3'

### - Amplification of the Env gene of patients

### • First amplification:

5' TATGAAACTTACGGGGATACTTGGG 3' (position 5697 - 5721 of the pNL4.3)
5' CTGCCAATCAGGGAAGTAGCCTTGTGT 3' (position 9135 - 9161 of the pNL4.3)

### • Nested-PCR:

(XbaI target)
   5' GTAGCAATAATAATAGCTCTAGAGCTGTGGTCCATAGTAATC 3' (position 6097 - 6138 of pNL4.3)
(Not I target)
   5' TACTTTTTGCGGCCGCGCCACCCATCTTATAGC 3' (position 8779 - 8811 of the pNL4.3)

### (D) HIV-based recombinant viral clones:

The procedure set out in the above section using primers, probes, target sequences, cell lines and stated conditions has permitted the following viral clones of the present invention to be obtained:
**IP HIV NL Ren: Deposit Number CECT 5842**
**IP HIV NL LacZ/pr Ren: Deposit Number CECT 5846**
**IP HIV NL LacZ/rt Ren: Deposit Number CECT 5845**
**IP HIV NL LacZ/pol Ren: Deposit Number CECT 5847**
**IP HIV NL LacZ/gag-pr Ren: Deposit Number CECT 5848**
**IP HIV NL LacZ/env Ren: Deposit Number CECT 5844**
**IP HIV JRRen: Deposit Number CECT 5843**

### 2.- EVALUATION OF THE VIRAL CLONES OF THE INVENTION IN DIFFERENT SYSTEMS OF ANALYTICAL DETERMINATION

### 2.1.- DETERMINATION SYSTEM FOR PHENOTYPIC RESISTANCES TO ANTIRETROVIRAL DRUGS

The tested clones were the following
**IP HIV NL Ren,**
**IP HIV NL LacZ/pol Ren,**
**IP HIV NL LacZ/pr Ren,**
**IP HIV NL LacZ/rt Ren,**
**IP HIV NL LacZ/gag-pr Ren and**
**IP HIV NL LacZ/env Ren**

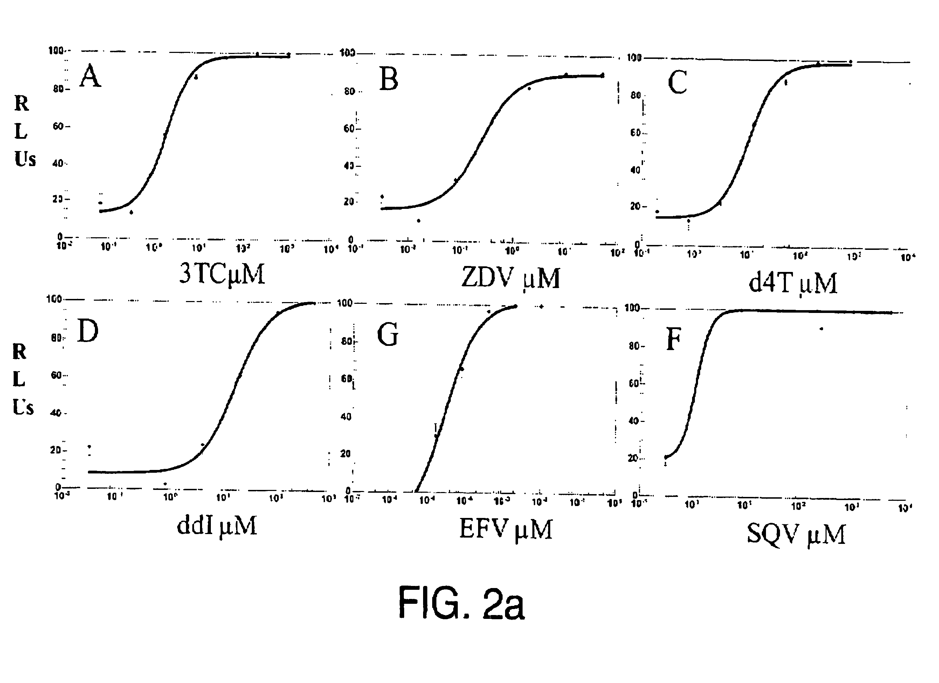

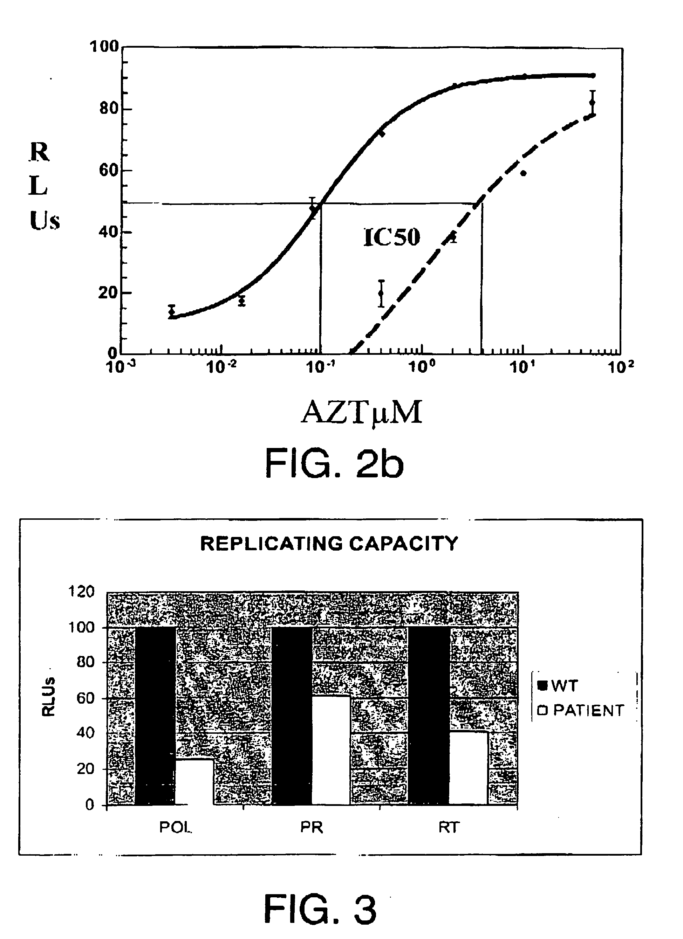

The results of the tests carried out with these viral clones according to the inventive system for the determination of phenotypic resistances to antiretroviral drugs are shown in figures 2a and 2b.

Figure 2a represents the phenotypic profile of sensitivity of the viral clone **IP HIV NL Ren** towards the following drugs: inhibitors of reverse transcriptase analogous to 3TC nucleosides (A), AZT/ZDV (B), d4T (C), ddI (D), inhibitors of reverse transcriptase not analogous to nucleosides; Efavirenz (E); inhibitors of protease: Saquinavir (F).

Figure 2b is a graphic representation illustrating a study of the determination of AZT resistance in a wild type virus (solid line) and in a virus with the mutations M41L, K07R, T215F, K219Q (broken line): Fold = 36.

Among the advantages of this system compared to other systems currently in existence, the following can be mentioned:
a. Possibility of separately analysing the resistance to inhibitors of protease and of reverse transcriptase. This makes it possible to conduct an independent evaluation of resistances to different pharmacological groups.
b. Greater efficacy in the evaluation of viral isolates with low replicative capacity.
c. It permits monitoring of certain patients in therapeutic failure.
d. With regard to the system patented by Virologic (US patent 5,837,464), the system of recombinant viral clones of the present invention has notable differences, leading to the important advantages cited earlier, namely:
   - Virologic clones the luciferase gene in the envelope and the applicant in Nef.
   - Virologic uses similar but not identical enzymes to those used here.
   - The system of the present invention has modified the NL4.3 skeleton by mutagenesis.
   - In the present invention, multiple cycle vectors and separate cloning of the RT and Protease, and evaluation of the gag-Protease and gag-pol fragments can be used, aspects which the Virologic system does not permit.

### 2.2.- DETERMINATION SYSTEM OF THE REPLICATIVE CAPACITY

Figure 3 represents a histogram showing the improvement in the recovery of a virus with multiple resistance mutations in the Protease and RT when separate cloning is carried out of both fragments than with the complete pol gene. This effect is due to the accumulation of loss of viral fitness which can result in viruses with low replicative capacity that are difficult to detect in single cycle tests when the loss of fitness owing to mutations in the Reverse Transcriptase and Protease are added together.

The viral clones submitted for evaluation were the following
**IP HIV NL LacZ/pol Ren,**
**IP HIV NL LacZ/pr Ren,**
**IP HIV NL LacZ/rt Ren and**
**IP HIV NL LacZ/gag-pr Ren**

The most outstanding advantages of the inventive system compared to others currently in existence are the following:
a. The system is very sensitive since it uses renilla activity.
b. The system directly measures antiviral activity, unlike the MTT test which measures protection against the cytopathic effect, which is an indirect measurement of viral replication.
c. It has the possibility of separately cloning reverse transcriptase or protease, which permits it to define in which protein the loss of replicative capacity lies.
d. It has the possibility of jointly cloning the gag-pro gene which permits a definition to be made of the role of excision sites in the polyprotein of the viral core by the protease of HIV in improving viral replicative capacity.
e. The use of viral systems in which replication can be detected with a limited number of cycles means that, when viral escape exists, the neutralisation curves in multiple cycles of the virus are not equalised.

### 2.3.- DETERMINATION SYSTEM OF VIRAL TROPISM, PHENOTYPIC RESISTANCES TO FUSION INHIBITORS

The proposed invention is based on the system of cloning gene fragments of the envelope in carrier viral vectors of marker genes. A cell is required which expresses at the same time the two largest coreceptors of the virus CCR5 and CXCR4.

### (A) General description of the technique.

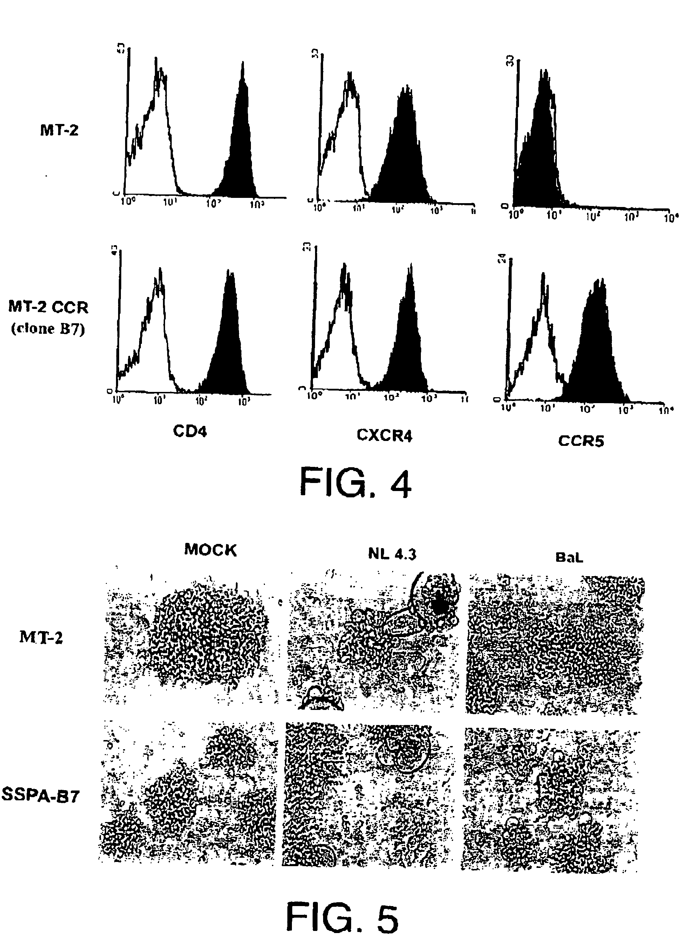

Starting from 0.5 ml of the patient's plasma, the extraction of RNA from the HIV is carried out.
- The viral RNA is retrotranscribed and then amplified using primers for each viral gene by means of chain reaction of the polymerase. The primers include specific restriction sites for later cloning in the reference virus and include the entire envelope of the virus.
- Following enzymatic digestion of the amplificate and of the reference virus, an *in vitro* ligation process is carried out using the T4 ligase.
- The population of the generated recombinant provirus is transfected in the cell line 293-T and acts as a producer cell of recombinant viruses.
- The infectious progeny of recombinant viruses is gathered 48 hours after the transfection and is used for infecting the cell line SSPA-B7 which expresses CCR5 and CXCR4 (Figure 4)

### (B) Virus:

This starts from the proviral vector NL4.3 (Adachi et al. 1986). These clones have been genetically modified in the laboratory producing multiple cycle viral clones and in which the complete env gene is cloned. With the generated recombinant virus, viral tropism or the resistance of the entry to inhibitors can be analysed. The corresponding viral clones are the following:
**IP HIV NL Ren,**
**IP HIV NL JRRen and**
**IP HIV NL LacZ/env Ren**

### (C) Cells

A cellular clone of SSPA-B7 has been generated by means of genetic engineering techniques which expresses the receptor CCR5 (Figure 4) and which is susceptible to infection by the virus R5, X4 or R5X4. Infection by these three variants is productive and induces cytopathic effect (Figure 5).

The most outstanding advantages of the inventive system compared to others currently in existence are the following:
a. The possibility of cloning the complete envelope of HIV. Other systems use recombination which presents a very low efficacy or they clone smaller fragments of the envelope.
b. The availability of a cell which expresses receptors CCR5 and CXCR4.
c. Its use in phenotypic tests on entry inhibitors.

### 2.4.- SYSTEM FOR DETECTION AND TITRATION OF NEUTRALISING ANTIBODIES

### (A) General description of the technique.

The proposed invention is based on the measurement of the neutralising activity in patients' serum against infection of a permissive line of marker gene carrier viruses and with different envelopes. The system includes viral clones with envelopes R5 and X4 and a cell which expresses the two largest coreceptors of the virus CCR5 and CXCR4.

### (B) Virus:

This starts from the proviral vector NL4.3 (Adachi et al. 1986). These clones have been genetically modified in the laboratory producing multiple cycle viral clones in which the complete env gene is cloned. With the generated recombinant virus one can analyse the neutralising capacity against different envelopes of the virus including that of the patient's own virus. The corresponding viral clones thus obtained and evaluated were the following
**IP HIV NL Ren,**
**IP HIV NL JRRen and**
**IP HIV NL LacZ/env Ren**

### (C) Cells

A SSPA-B7 cellular clone has been generated by means of genetic engineering techniques which expresses the receptor CCR5 (Figure 4) and which is susceptible to infection by the virus R5, X4 or R5X4. Infection by these three variants is productive and induces cytopathic effect (Figure 5).

### (D) Results

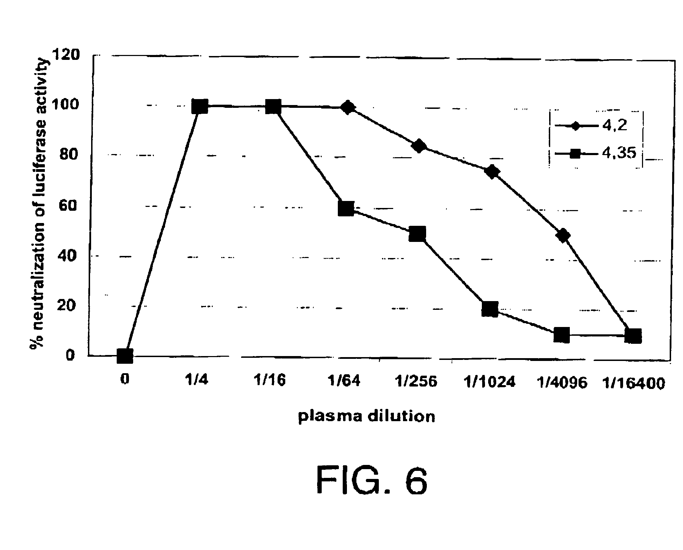

The results of the tests conducted with these viral clones according to the inventive system for the detection and titration of neutralising antibodies are illustrated in figure 6.

Said figure 6 is a graphic representation showing the results of the analysis of the neutralising capacity of HIV NL Ren virus of a patient's plasma before (4.35) and after (4.2) conducting a series of controlled treatment interruptions. In the classic MTT test, the differences between the two samples could not be observed.

Among the advantages of the system compared to others currently in existence, the following have to be highlighted:
a. The system is very sensitive since it uses renilla activity.
b. The system directly measures antiviral activity, unlike the MTT test which measures protection against the cytopathic effect, which is an indirect measurement of viral replication.
c. It has the possibility of cloning the complete envelope of different HIVs or even that of the patient himself (autologous neutralisation test).
d. The use of viral systems in which replication can be detected with a limited number of cycles means that, when viral escape exists, the neutralisation curves in multiple cycles of the virus are not equalised.
e. The availability of a cell which expresses the receptors CCR5 and CXCR4.
f. The renewed interest in studying neutralising antibodies in the context of the new vaccine models and their use as surrogate marker which will increase the demand for these tests in the immediate future.
g. The system is robotisable.

### 2.5.- SYSTEM FOR SCREENING COMPOUNDS AND PRODUCTS HAVING POTENTIAL ACTIVITY AGAINST HIV

### (A) General description of the technique.

The proposed invention is based on the measurement of antiviral activity against HIV of chemical compounds and derivatives of natural products using marker gene carrier viruses.

### (B) Virus:

This starts from the proviral vector NL4.3 (Adachi et al. 1986). These clones have been genetically modified in the laboratory producing multiple cycle viral clones with the envelope of HIV.

By limiting the infection to a singe replication cycle (18 h), antiviral activity can be detected from the entry process up to the transcription/translation of viral proteins. In this period of time, antiviral action in later stages, as in the case of protease inhibitors or viral encapsidating or gemmation inhibitors, would not be detected.

For these cases, renilla activity beyond the first cycle (18 hours) is evaluated. A drop in the luciferase activity in the single and multiple cycle indicates that the compound acts in stages prior to the processing of viral proteins. Nevertheless, if it only acts on the multiple cycle, this would indicate that it acts in post-integration/viral replication stages. The corresponding recombinant viral clone is as follows:
**IP HIV NL Ren**

### (C) Results

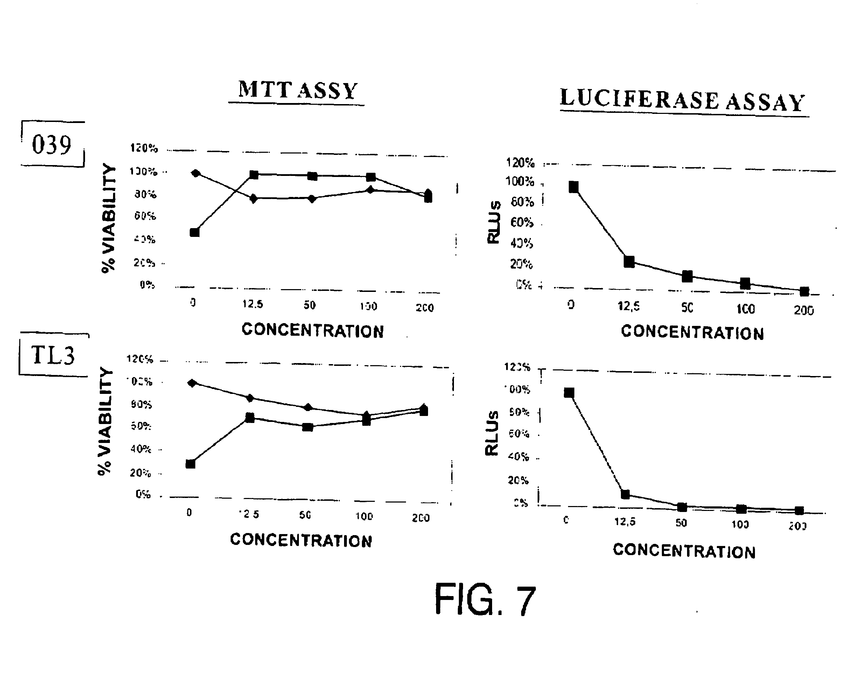

The results of the tests conducted with these viral clones according to the inventive system for the screening of compounds are illustrated in figure 7, where the graphic representations are shown corresponding to the analysis of antiviral activity of two compounds derived from plant products. In the classic MTT test, the toxicity of the compound (line with diamonds) and the protection against the cytopathic effect (lines with squares) are measured. The panels on the right analyse the inhibition of the replication of a luciferase virus. The mechanism of action of both compounds is being characterised at this moment and we know that compound 039 is a viral entry inhibitor.

The following advantages of the system can be highlighted compared to others currently in existence:
a. No antiviral activity evaluation systems have been described using recombinant viruses.
b. The system is very sensitive since it uses renilla activity.
c. The system directly measures antiviral activity, unlike the MTT test which measures protection against the cytopathic effect, an indirect measure of viral replication.
d. The system is robotisable and applicable to mass screening.

## Claims

1. HIV-based recombinant viral clones, **characterised in that** they comprise the *LacZ* gene cloned by substituting a sequence of the genome, and **in that** they comprise a general structure that contains the following elements in 5' to 3' direction,
- LTR or redundant terminal sequences (R) which contains numerous consensus sequences for transcription factors that regulate viral expression;
- *gag* gene;
- *pol* gene whose 5' end overlaps with *gag* element;
- *vif* gene whose 5' end overlaps with *pol* element and 3' end overlaps *vpr* clement;
- *vpr* gene whose 5' end overlaps *vif* element;
- *tat* whose second exon is contained inside *env* sequence;
- *vpu* gene;
- *env* gene ;
- *rev* genewhose second exon is contained inside *env* sequence;
- *nef* is the gene that codes protein Nef, and is truncated at the bases in positions 8796 and 8887 of the viral genome;
- NotI is a restriction site for NotI enzyme, that has been introduced by directed mutagenesis at position 8796 of the viral genome;
- XhoI is a restriction site for the XhoI enzyme, in position 8887 of the viral genome;
- Renilla is the gene that codes the luciferase reporter protein Renilla, and that has been cloned in restriction sites NotI-XhoI in position 5' and 3', respectively; and
- LTR, whose 5' end overlaps with the 3' end of *nef* element.

2. Recombinant viral clone according to claim 1, **characterised in that** said clone is the clone IP HIV NL LacZ/rt Ren, deposited in the Spanish collection of Type Cultures as CECT 5845, which possesses a unique restriction site for enzyme NcoI that has been introduced by directed mutagenesis at the position 2593 of the DNA sequence, and the *LacZ* gene is cloned in NcoI-AgeI restriction sites in positions 5' and 3', respectively, substituting the fragment of *pol* gene that codes the reverse transcriptase.

3. Recombinant viral clone according to claim 1, **characterised in that** said clone is the clone IP HIV NL LacZ/pr Ren, deposited in the Spanish Collection of Type Cultures as CECT 5846, which possesses a unique restriction site for NcoI enzyme introduced by directed mutagenesis in position 2593 of the DNA sequence, and *LacZ* gene is cloned between restriction sites ApaI-NcoI in positions 5' and 3', respectively, substituting the fragment of *pol* gene that encodes the protease.

4. Recombinant viral clone according to claim 1, **characterised in that** said clone is the clone IP HIV NL Lacz/pol Ren, deposited in the Spanish Collection of Type Cultures as CECT 5847, which possesses the *LacZ* gene cloned between restriction sites ApaI-AgeI in positions 5' and 3', respectively, substituting the fragment of *pol* gene that encodes the protease and the reverse transcriptase.

5. Recombinant viral clone according to claim 1, **characterised in that** said clone is the clone IP HIV NL LacZ/gag-pr Ren, deposited in the Spanish Collection of Type Cultures as CECT 5848, which possesses unique restriction sites for enzymes NarI and KspI, this last one introduced by directed mutagenesis, at positions 637 and 4498, respectively, in the DNA sequence, and *LacZ* gene is cloned between the restriction sites ApaI-NcoI in positions 5' and 3', respectively, substituting the fragment of *pol* gene that encodes the protease.

6. Recombinant viral clone according to claim 1, **characterised in that** said clone is the clone IP HIV NL Lacz/env Ren, deposited in the Spanish Collection of Type Cultures as CECT 5844, which possesses a unique restriction site for XbaI enzyme, introduced by directed mutagenesis in position 6112 of the DNA sequence, and *LacZ* gene is cloned between restriction sites XbaI-NotI in positions 5' and 3', respectively, substituting *env* gene.

7. Use of recombinant viral clones defined in any of claims 1 to 6, in analytical methods *in vitro* for the determination of phenotypic resistances to antiretroviral drugs for the treatment of HIV infection.

8. Use of recombinant viral clones defined in any of claims 1 to 6, in analytical methods *in vitro* for the determination of the replicative capacity of recombinant viruses carrying *gag*, *pol* and/or *env* sequences of patients with HIV infection.

9. Use of recombinant viral clones defined in claim 6, in analytical methods *in vitro* for the characterisation of viral tropism in HIV infection.

10. Use of recombinant viral clones defined in any of claims 1 to 6, in analytical methods *in vitro* for the detection of neutralising antibodies against Hlv in the serum of seropositive patients for HIV and non-infected individuals subjected to vaccination

11. use of recombinant viral clones defined in any of claims 1 to 6, in analytical methods *in vitro* for the screening and characterisation of compounds for antiviral activity towards HIV.

## Patentansprüche

1. Auf HIV basierende, rekombinante virale Klone, **dadurch gekennzeichnet, dass** sie das *LacZ*-Gen enthalten, das durch Substituieren einer Sequenz des Genoms kloniert wird, und **dadurch**, dass sie eine allgemeine Struktur umfassen, welche die folgenden Element in 5' - 3' Richtung enthält:
- LTR oder redundante terminale Sequenzen (R), die zahlreiche Konsensussequenzen für Transkriptionsfaktoren enthalten, welche eine virale Expression regulieren;
- *gag*-Gen;
- *pol*-Gen, dessen 5'-Ende mit einem *gag*-Element überlappt;
- *vif*-Gen, dessen 5'-ende mit einem *pol*-Element überlappt und dessen 3'-Ende ein *vpr*-Element überlappt;
- *vpr*-Gen, dessen 5'-Ende ein *vif*-Element überlappt;
- *tat*, dessen zweites Exon innerhalb einer *env*-Sequenz enthalten ist;
- *vpu*-Gen;
- *env*-Gen;
- *rev*-Gen, dessen zweites Exon innerhalb der *env*-Sequenz enthalten ist;
- *nef* ist das Gen, welches das Nef-Protein kodiert, und ist an den Basen an den Positionen 8796 und 8887 des viralen Genoms verkürzt;
- NotI ist eine Restriktionsstelle für ein NotI-Enzym, das durch direkte Mutagenese an der Position 8796 des viralen Genoms eingeführt worden ist;
- XhoI ist eine Restriktionsstelle für das XhoI-Enzym an der Position 8887 des viralen Genoms;
- Renilla ist das Gen, welches das Renilla-Luciferase-Reporterprotein kodiert, und das in die Restriktionsstellen NotI-XhoI an der Position 5' bzw, 3' kloniert wurde; und
- LTR, dessen 5'-Ende mit dem 3'-Ende des *nef*-Elements überlappt.

2. Rekombinanter viraler Klon nach Anspruch 1, **dadurch gekennzeichnet, dass** der Klon der Klon IP HIV NL LacZ/rt Ren ist, der bei der *Coleccción Espanola de Cultivos Tipo* ("Spanish Collection of Type Cultures") als CECT 5845 hinterlegt ist, der eine einzigartige Restriktionsstelle für das NcoI-Enzym aufweist, das durch direkte Mutagenese an der Position 2593 der DNA-Sequenz eingeführt wird, und dass das *LacZ*-Gen in NcoI-AgeI-Restriktionsstellen an den Positionen 5' bzw. 3' kloniert ist, wodurch das Fragment des *pol*-Gens, das die reverse Transkriptase kodiert, substituiert ist.

3. Rekombinanter viraler Klon nach Anspruch 1, **dadurch gekennzeichnet, dass** der Klon der Klon IP HIV NL LaCZ/pr Ren ist, der bei der *Colección Espanola de Cultivos Tipo* als CECT 5846 hinterlegt ist, der eine einzigartige Restriktionsstelle für das NcoI-Enzym aufweist, das durch direkte Mutagenese an der Position 2593 der DNA-Sequenz engeführt wird, und dass das *LacZ*-Gen zwischen Restriktionsstellen ApaI-NcoI an den Positionen 5' bzw. 3' kloniert ist, wodurch das Fragment des *pol*-Gens, das die Protease kodiert, substituiert ist.

4. Rekombinanter viraler Klon nach Anspruch 1, **dadurch gekennzeichnet, dass** der Klon der Klon IP HIV NL LacZ/pol Ren ist, der bei der *Coleccción Espanola de Cultivos Tipo* als CECT 5847 hinterlegt ist, der das *LaZ*-Gen enthält, das zwischen Restriktionsstellen ApaI-AgeI an den Positionen 5' bzw. 3' kloniert ist, wodurch das Fragment des *pol*-Gens substituiert ist, das die Protease und die reverse Transkriptase kodiert.

5. Rekombinanter viraler Klon nach Anspruch 1, **dadurch gekennzeichnet, dass** der Klon der Klon IP HIV NL LacZ/gag-pr Ren ist, der bei der *Colección Espanola de Cultivos Tipo* als CECT 5848 hinterlegt ist, der einzigartige Restriktionsstellen für die NarI- und KspI-Enzyme aufweist, wobei letzteres durch direkte Mutagenese an den Positionen 637 bzw. 4498 in die DNA-Sequenz eingeführt wird, und dass das *LacZ*-Gen zwischen den Restriktionsstellen ApaI-NcoI an den Positionen 5' bzw. 3' kloniert ist, wodurch das Fragment des *pol*-Gens, welches die Protease kodiert, substituiert sind.

6. Rekombinanter viraler Klon nach Anspruch 1, **dadurch gekennzeichnet, dass** der Klon der Klon IP HIV NL LacZ/env Ren ist, der bei der *Colección Espanola de Cultivos Tipo* als CECT 5844 hinterlegt ist, der eine einzigartige Restriktionsstelle für das XbaI-Enzym aufweist, eingeführt durch direkte Mutagenese an der Position 6112 der DNA-Sequenz, und dass das *LacZ*-Gen zwischen den Restriktionsstellen XbaI-NotI an den Positionen 5' bzw. 3' kloniert ist, wodurch das *env*-Gen substituiert ist.

7. Verwendung von rekombinanten viralen Klonen nach einem der Ansprüche 1 bis 6 in *in*-*vitro*-Analyseverfahren für die Bestimmung von phänotypischen Resistenzen gegenüber antiretroviralen Arzneimitteln zur Behandlung einer HIV-Infektion.

8. Verwendung von rekombinanten viralen Klonen nach einem der Ansprüche 1 bis 6 in *in*-*vitro*-Analyseverfahren für die Bestimmung der Replikationsfähigkeit von rekombinanten Viren, welche die *gag*-, *pol-*und/oder *env*-Sequenzen tragen, von Patienten mit HIV-Infektion

9. Verwendung von rekombinanten viralen Klonen nach Anspruch 6 in *in-vitro-*Analyseverfahren für die Charakterisierung eines viralen Tropismus bei HIV-Infektion.

10. Verwendung von rekombinanten viralen Klonen nach einem der Ansprüche 1 bis 6 in *in*-*vitro*-Analyseverfahren für die Erfassung von neutralisierenden Antikörpern gegen HIV im Serum von seropositiven Patienten bei HIVinfizierten und nicht-infizierten Individuen, die einer Impfung unterzogen wurden.

11. Verwendung von rekombinanten viralen Klonen nach einem der Ansprüche 1 bis 6 in *in*-*vitro*-Analyseverfahren für die Durchmusterung und Charakterisierung von Verbindungen für eine gegen HIV gerichtete antivirale Wirkung.

## Revendications

1. Clones viraux recombinés à base de HIV, **caractérisés en ce qu'**ils comprennent le gène LacZ, cloné en remplaçant une séquence du génome, et **en ce qu'**ils comprennent une structure générale qui contient les éléments suivants en direction 5' vers 3' :
- des séquences LTR ou terminales redondantes (R), qui contiennent plusieurs séquences consensus pour les fauteurs de transcription qui régulent l'expression virale ;
- le gène gag ;
- le gène pol dont l'extrémité 5' chevauche l'élément gag ;
- le gène vif dont l'extrémité 5' chevauche l'élément pol et l'extrémité 3' chevauche l'élément vpr ;
- le gène vpr dont l'extrémité 5' chevauche l'élément vif ;
- taL dont le deuxième exon est contenu à l'intérieur de la séquence env ;
le gène vpu ;
- le gène env ;
- le gène rev dont le deuxième exon est contenu à l'intérieur de la séquence env ;
- nef est le gène qui code la protéine Nef, et est tronqué aux bases en les positions 8796 et 8887 du génome viral ;
NotI est un site de restriction pour l'enzyme NotI, qui a été introduit par mutagenèse dirigée à la position 8796 du génome viral ;
- XhoI est un site de restriction pour l'enzyme XhoI, qui a été introduit par mutagenèse dirigée à la position 8887 du génome viral ;
- Renilla est le gène qui code la protéine rapporteur luciférase Renilla, et qui a été cloné dans les sites de restriction NotI-XhoI en les positions 5' et 3', respectivement, et
- une séquence LTR, dont l'extrémité 5' chevauche l'extrémité 3' de l'élément nef.

2. Clone viral recombine selon la revendication 1, **caractérisé en ce que** ledit clone est le clone IP HIV NL LacZ/rt Ren, déposé, au Spanish Collection of Type Cultures en tant que CECT 5845, qui possède un site unique de restriction pour l'enzyme NcoI, qui a été introduit par mutagenèse directe en la position 2593 de la séquence d'ADN, et le gène LacZ est cloné entre les sites de restriction NcoI-AgeI en les positions 5' et 3', respectivement, pour remplacer le fragment du gène pol qui code la transcriptase réverse.

3. Clone viral recombiné selon la revendication 1, **caractérisé en ce que** ledit clone est le clone IP HIV NL LacZ/pr Ren, déposé au Spanish Collection of Type Cultures en tant que CECT 5846, qui possède un site unique de restriction pour l'enzyme NcoI, qui a été introduit par mutagenèse directe en la position 25H3 de la séquence d'ADN, et le géne LacZ est clone entre les sites de restriction ApaI-NcoI en les positions 5' et 3', respectivement, pour remplacer le fragment du gène pol qui code la protéase.

4. Clone viral recombine selon la revendication 1, **caractérisé en ce que** ledit clone est le clone IP HIV NL LacZ/pol Ren, déposé au Spanish Collection of Type Cultures en tant que CECT 5847, qui possède le gène LacZ cloné entre les sites de restriction ApaI-AgeI en les positions 5' et 3', respectivement, pour remplacer le fragment du gène pol qui code la protéase et la transcriptase reverse.

5. Clone viral recombine selon La revendication 1, **caractérisé en ce que** ledit clone est le clone IP HIV NL LacZ/gag-pr Ren, déposé au Spanish Collection of Type Cultures en tant que CECT 5848, qui possède les sites uniques de restriction pour les enzymes NarI et KapI, ce dernier introduit par mutagenèse directe, en les positions 637 et 4498, respectivement, de la séquence d'ADN, et le géne Lacs est cloné entre les sites de restriction ApaI-NcoI en les positions 5' et 3', respectivement, pour remplacer le fragment du gène pol qui code la protéase.

6. Clone viral recombiné selon la revendication 1, **caractérise en ce que** ledit clone est le clone IF HIV NL LacZ/env Ren, déposé au Spanish Collection of Type Cultures en tant que CECT 5844, qui possède un site unique de restriction pour l'enzyme XbaI, introduit par mutagenèse directe en la position 6112 de la séquence d'ADN, et le géne LacZ est clone entre les sites de restriction XbaI-NotI en les positions 5' et 3', respectivement, pour remplacer lie géne env.

7. Utilisation des clones viraux recombinés définis dans l'une quelconque des revendications 1 à 6, dans des procédés analytiques *in vitro* pour la détermination des résistances phénotypiques aux médicaments antirétroviraux pour le traitement d'une infection au HIV.

8. Utilisation des clones viraux recombinés définis dans l'une quelconque des revendications 1 à 6, dans des procédés analytiques *in vitro* pour la détermination de la capacité de réplication des virus recombinés portant les séquences gag, pol et/ou env chez des patients infectés par le HIV,

9. Utilisation des clones viraux recombinés selon la revendication 6, dans des procédés analytiques *in vitro* pour la caractérisation du tropisme viral dans une infection au HIV.

10. Utilisation des clones viraux recombinés définis dans l'une quelconque des revendications 1 à 6, dans des procédés analytiques *in vitro* pour la détection d'anticorps neutralisants contre le HIV. dans le sérum de patients positifs au HIV et d'individus non infectés, mais vaccinés.

11. Utilisation des clones viraux définis dans l'une quelconque des revendications 1 à 6, dans des procédés analytiques *in vitro* pour le criblage et la caractérisation de composés ayant une activité antivirale contre le HIV.


### SEQUENCE LIST

<110> FUNDACION PARA LA INVESTIGACIÓN Y LA PREVENCIÓN DEL STDA EN ESPAÑA; INSTITUTO DE SALUD CARLOS III
<130> NOVEL HIV BASED RECOMBINANT VIRAL CLONES AND USE THEREOF IN ANALYTICAL METHODS
<130> 120050086MAD
<160> 24
<170> PatentIn version 3.1
<210> 1
   <211> 43
   <212> DNA
   <213> Artificial
<220>
   <221> misc_feature
   <222> (1)..(43)
   <223> oligo mutagenesis NotI 1
<400> 1
   gctataagat gggtggcgcg gccgcaaaaa gtagtgtgat tgg 43
<210> 2
   <211> 43
   <212> DNA
   <213> Artificial
<220>
   <221> misc feature
   <222> (1)..(43)
   <223> oligo mutagenesis NotI 2
<400> 2
   ccaatcacac tactttttgc ggccgcgcca cccatcttat agc 43
<210> 3
   <211> 37
   <212> DNA
   <213> Artificial
<220>
   <221> misc feature
   <222> (1).. 37)
   <223> oligo mutagenesis NcoI 1
<400> 3
   ccagtaaaat taaagccagc catggatggc ccaaaag 37
<210> 4
   <211> 37
   <212> DNA
   <213> Artificial
<220>
   <221> misc_feature
   <222> (1)..(37)
   <223> oligo mutagenesis NcoI 2
<400> 4
   cttttgggcc atccatggct ggctttaatt ttactgg 37
<210> 5
   <211> 38
   <212> DNA
   <213> Artificial
<220>
   <221> misc_feature
   <222> (1)..(38)
   <223> oligo mutagenesis KspI 1
<400> 5
   gaagcagaag taattcccgc ggagacaggg caagaaac 38
<210> 6
   <211> 38
   <212> DNA
   <213> Artificial
<220>
   <221> mis_feature
   <222> (1).. (38)
   <223> oligo mutagenesis KspI 2
<400> 6
   gtttcttgcc ctgtctccgc gggaattact tctgcttc 38
<210> 7
   <211> 36
   <212> DNA
   <213> Artificial
<220>
   <221> misc_feature
   <222> (1)..(36)
   <223> oligo mutagenesis Narl 1
<400> 7
   gaaaataccg catcaggacc cattcgccat tcaggc 36
<210> 8
   <211> 36
   <212> DNA
   <213> Artificial
<220>
   <221> misc_feature
   <222> (1)..(36)
   <223> oligo mutagenesis NarI 2
<400> 8
   gcctgaatgg cyaatgggtc ctgatgcggt attttc 36
<210> 9
   <211> 55
   <212> DNA
   <213> Artificial
<220>
   <221> misc_feature
   <222> (1) .. (55)
   <223> oligo mutagenesis XbaI 1
<400> 9
   gcattagtag tagcaataat aatagctcta gagctgtggt ccatagtaat catag 55
<210> 10
   <211> 55
   <212> DNA
   <213> Artificial
<220>
   <221> misc_feature
   <222> (1) .. (55)
   <223> oligo mutagenesis XbaI 2
<400> 10 
ctatgattac tatggaccac agctctagag ctattattat tgctactact
aatgc 55
<210> 11
   <211> 25
   <212> DNA
   <213> Artificial
<220>
   <221> misc_feature
   <222> (1) .. (25)
   <223> oligo POL 1
<400> 11
   gccaaaaatt gcagggcccc tagga 25
<210> 12
   <211> 35
   <212> DNA
   <213> Artificial
<220>
   <221> misc_feature
   <222> (1) .. (35)
   <223> oligo POL 2
<400> 12
   tcttttgatg ggtcataata cactccatgt accgg 35
<210> 13
   <211> 25
   <212> DNA
   <213> Artificial
<220>
   <221> misc_feature
   <222> (1) .. (25)
   <223> oligo PRO 1
<400> 13
   gccaaaaatt gcagggcccc tagga 25
<210> 14
   <211> 36
   <212> DNA
   <213> Artificial
<220>
   <221> misc_feature
   <222> (1) .. (36)
   <223> oligo PRO 2
<400> 14
   catgccatgg ctggctttaa ttttactggt acagtc 36
<210> 15
   <211> 35
   <212> DNA
   <313> Artificial
<220>
   <221> misc_teature
   <222> (1) .. (35)
   <223> oligo RT 1
<400> 15
   catgccatgg atggcccaaa agttaaacaa tggcc 35
<210> 16
   <211> 35
   <212> DNA
   <213> Artificial
<220>
   <221> misc_feature
   <222> (1) .. (35)
   <223> oligo RT 2
<400> 16
   Lcttttgatg ggtcataata cactccatgt accgg 35
<210> 17
   <211> 30
   <212> DNA
   <213> Artificial
<220>
   <221> misc_feature
   <222> (1) .. (30)
   <223> oligo GAG-PR 1
<400> 17
   ggaaaatctc tagcagtggc gcccgaacag 30
<210> 18
   <211> 36
   <212> DNA
   <213> Artificial
<220>
   <221> misc_feature
   <222> (1) .. (36)
   <223> oligo CAC-PR 2
<400> 18
   catgccatgg ctggctttaa ttttactggt acagtc 36
<210> 19
   <211> 30
   <212> DNA
   <213> Artificial
<220>
   <221> misc_feature
   <222> (1) .. (30)
   <223> oligo GAG POL 1
<400> 19
   ggaaaatctc tagcagtggc gcccgaacag 30
<210> 20
   <211> 31
   <212> DNA
   <213> Artificial
<220>
   <221> misc feature
   <222> (1)..(31)
   <223> oligo CAC-POL 2
<400> 20
   cttgccctgt ctctgctgga attacttctg c 31
<210> 21
   <211> 25
   <212> DNA
   <213> Artificial
<220>
   <221> misc_feature
   <222> (1) .. (25)
   <223> oligo Env 1
<400> 21
   tatgaaactt acggggatac ttggg 25
<210> 22
   <211> 27
   <212> DNA
   <213> Artificial
<220>
   <221> misc_feature
   <222> (1) .. (27)
   <223> oligo Env 2
<400> 22
   ctgccaatca gggaagtag cttgtgt 27
<210> 23
   <211> 42
   <212> DNA
   <213> Artificial
<220>
   <221> misc feature
   <222> (1) .. (42)
   <223> oligo nested Env 1
<400> 23
   gtagcaataa taatagctct agagctgtgg tccatagtaa tc 42
<210> 24
   <211> 33
   <212> DNA
   <213> Artificial
<220>
   <221> misc_feature
   <222> (1) .. (33)
   <223> oligo nested Env 2
<400> 24
   tactttttgc ggccgcca cccatcttat agc 33